# EUROPEAN PATENT SPECIFICATION

(11) **EP 2 849 852 B1**
(45) Date of publication and mention of the grant of the patent: **18.09.2019**
(21) Application number: 13724345.7
(22) Date of filing: 10.05.2013
(51) Int. Cl.: A61K 35/763, A61P 35/00

(54) **HERPES SIMPLEX VIRUS FOR THE TREATMENT OF LIVER CANCER**
HERPES SIMPLEX VIRUS ZUR BEHANDLUNG VON LEBERKREBS
VIRUS DE L'HERPES SIMPLEX POUR LE TRAITEMENT DU CANCER DU FOIE

(30) Priority: 11.05.2012 EP 12167750; 11.05.2012 US 201261645785 P
(43) Date of publication of application: 25.03.2015
(73) Proprietor: Virttu Biologics Limited, Glasgow G51 4WF (GB)
(72) Inventor: CONNER, Joe, Glasgow G51 4WF (GB)
(74) Representative: Hambleton, Bernadette Angelina
(86) International application number: PCT/GB2013/051217
(87) International publication number: WO 2013/167909

(56) References cited:
- WO-A1-2007/026146
- US-A1- 2010 272 691
- LAM PAULA Y P ET AL: "An efficient and safe herpes simplex virus type 1 amplicon vector for transcriptionally targeted therapy of human hepatocellular carcinomas", MOLECULAR THERAPY, ACADEMIC PRESS, SAN DIEGO, CA, US, vol. 15, no. 6, 1 June 2007 (2007-06-01), pages 1129-1136, XP002606531, ISSN: 1525-0016
- REINBLATT ET AL: "Herpes Viral Oncolysis: A Novel Cancer Therapy", JOURNAL OF THE AMERICAN COLLEGE OF SURGEONS, COLLEGE, CHICAGO, IL, US, vol. 205, no. 4, 1 October 2007 (2007-10-01), pages S69-S75, XP022283650, ISSN: 1072-7515, DOI: 10.1016/J.JAMCOLLSURG.2007.06.333
- HAWKINS L K ET AL: "Oncolytic biotherapy: a novel therapeutic platform", LANCET ONCOLOGY, LANCET PUBLISHING GROUP, LONDON, GB, vol. 3, no. 1, 1 January 2002 (2002-01-01) , pages 17-26, XP004811728, ISSN: 1470-2045, DOI: 10.1016/S1470-2045(01)00618-0
- TOMOKI TODO: "Oncolytic virus therapy using genetically engineered herpes simplex viruses", FRONTIERS IN BIOSCIENCE, vol. 13, no. 13, 1 September 2002 (2002-09-01), pages 2060-159, XP055009778, ISSN: 1093-9946, DOI: 10.2741/2823
- WONG R J ET AL: "Oncolytic herpesvirus effectively treats murine squamous cell carcinoma and spreads by natural lymphatics to treat sites of lymphatic metastases", HUMAN GENE THERAPY, MARY ANN LIEBERT, NEW YORK ,NY, US, vol. 13, 1 July 2002 (2002-07-01), pages 1213-1223, XP002971610, ISSN: 1043-0342, DOI: 10.1089/104303402320138998
- BROWN C W ET AL: "Oncolytic Viruses: A New Weapon to Fight Cancer", JOURNAL OF MEDICAL IMAGING AND RADIATION SCIENCES, ELSEVIER, AMSTERDAM, NL, vol. 39, no. 3, 1 September 2008 (2008-09-01), pages 115-127, XP025350702, ISSN: 1939-8654 [retrieved on 2008-09-07]
- RAFAELA ARGNANI ET AL: "Characterization of herpes simplex virus 1 strains as platforms for the development of oncolytic viruses against liver cancer", LIVER INTERNATIONAL, vol. 31, no. 10, 1 November 2011 (2011-11-01), pages 1542-1553, XP055037786, ISSN: 1478-3223, DOI: 10.1111/j.1478-3231.2011.02628.x
- VARGHESE SUSAN ET AL: "Oncolytic herpes simplex virus vectors for cancer virotherapy", CANCER GENE THERAPY, NORWALK, CT, US, vol. 9, no. 12, 1 December 2002 (2002-12-01), pages 967-978, XP002313119, ISSN: 0929-1903, DOI: 10.1038/SJ.CGT.7700537
- LI Y ET AL: "A hepatocellular carcinoma-specific adenovirus variant, CV890, eliminates distant human liver tumors in combination with doxorubicin", CANCER RESEARCH, AMERICAN ASSOCIATION FOR CANCER RESEARCH, US, vol. 61, no. 17, 1 January 2001 (2001-01-01), pages 6428-6436, XP002194693, ISSN: 0008-5472
- FALKSON ET AL: "Chemotherapy Studies in Primary Liver Cancer: A Prospective Randomized Clinical Trial", CANCER, AMERICAN CANCER SOCIETY, PHILADELPHIA, PA, US, vol. 42, no. 5, 1 November 1978 (1978-11-01), pages 2149-2156, XP002120029, ISSN: 0008-543X
- BRUIX J ET AL: "67 EFFICACY AND SAFETY OF SORAFENIB IN PATIENTS WITH HEPATOCELLULAR CARCINOMA (HCC): SUBANALYSIS OF SHARP TRIAL BASED ON BARCELONA CLINIC LIVER CANCER (BCLC) STAGE", JOURNAL OF HEPATOLOGY, MUNKSGAARD INTERNATIONAL PUBLISHERS, COPENHAGEN, DK, vol. 50, 1 April 2009 (2009-04-01), pages S28-S29, XP026495680, ISSN: 0168-8278, DOI: 10.1016/S0168-8278(09)60069-6 [retrieved on 2009-04-01]

## Description

### Field of the Invention

The present invention relates to the use of an oncolytic herpes simplex virus in the treatment of primary liver cancer.

### Background to the Invention

Hepatocellular carcinoma (HCC), also known as malignant hepatoma, is a primary liver cancer and one of the most common solid organ malignancies. It is thought to be the third most common cause of death due to cancer causing an estimated 662,500 deaths annually. Diagnosed HCC tripled in the period between 1975 and 2005. Patient prognosis following diagnosis of HCC is poor, with ∼90% patients not surviving beyond six months of diagnosis.

HCC is often associated with viral hepatitis B or C, alcoholic liver disease, cirrhosis, or haemochromatosis.

Existing treatments include surgery (e.g. hepatic resection or orthotopic liver transplantation), local ablative therapy (e.g. percutaneous ethanol injection), systemic chemotherapy (palliative), tumor embolisation and chemoembolistation (palliative).

Surgical resection represents the only realistic cure for HCC, although this procedure is limited by the nature of the tumor burden within the liver and the degree of liver function remaining after resection. Only about 10-15% of patients with HCC are suitable for surgical resection. Furthermore, instances of recurrence of tumor after surgical resection are high.

According to the EASL Panel of Experts on HCC "...none of the available options offers an unequivocal survival benefit to patients with intermediate-advanced HCC" (Bruix et al, 2001; J Hepatol 35:421-30).

Oncolytic virotherapy concerns the use of lytic viruses which selectively infect and kill cancer cells. Some oncolytic viruses are promising therapies as they display exquisite selection for replication in cancer cells and their self-limiting propagation within tumors results in fewer toxic side effects. Several oncolytic viruses have shown great promise in the clinic (Bell, J., Oncolytic Viruses: An Approved Product on the Horizon? Mol Ther. 2010; 18(2): 233-234).

Kelly et al and Geevarghese et al reported a Phase I/II trial of the safety and potential clinical efficacy of the HSV-1 strain F mutant NV1020 against liver metastasis of colorectal cancer (Kelly et al., Expert Opin Investig Drugs. 2008 July ; 17(7): 1105-1113; Geevarghese et al., HUMAN GENE THERAPY 21:1119-1128 (September 2010)). Patients enrolled in the trial had undergone partial hepatectomy and prior adjuvant chemotherapy (5-Fluorouracil (5-FU)). NV1020 was administered at doses of 3 x 10⁶, 1 x 10⁷, 3 x 10⁷, and 1 x 10⁸ by infusion to the hepatic artery. Patients received a chemotherapy infusion pump and cycles of floxuridine (FUDR) plus a continuation of the prior chemotherapy for 1 or 2 months after administration.

NV1020 is an attenuated, recombinant virus derived from the HSV-1 strain F mutant R7020, and was originally designed as a potential HSV-2 vaccine (Meignier B, Roizman B. Herpes simplex virus vaccines. Antiviral Res. 1985 Suppl 1:259-265; Meignier B, Longnecker R, Roizman B. In vivo behavior of genetically engineered herpes simplex viruses R7017 and R7020: construction and evaluation in rodents. J Infect Dis. 1988; 158(3):602-614).

NV1020 was attenuated by deletion of a 15-kb region at the UL/S junction encompassing only one copy of the diploid genes α0, α4, and γ₁34.5 encoding the proteins ICP0, ICP4, and ICP34.5, respectively, and one copy of UL56. NV1020 is further attenuated by a 700-bp deletion encompassing the thymidine kinase (TK) gene locus and the promoter for the gene UL24.

In addition to these deletions, NV1020 carries an exogenous inserted copy of the tk gene under restrictive control of the ICP4 promoter, and a 5.2-kb fragment of HSV-2 DNA. Both of these insertions are located at the UL/S junction (Wong RJ, Kim SH, Joe JK, et al. Effective treatment of head and neck squamous cell carcinoma by an oncolytic herpes simplex virus. J Am Coll Surg. 2001; 193(1):12-21).

Kulu et al (Cancer Gene Therapy (2013), 1-8: Concurrent chemotherapy inhibits herpes simplex virus-1 replication and oncolysis) indicate that cellular responses to chemotherapeutic agents provide an unfavorable environment for HSV-1-mediated oncolysis and indicates that this observation is relevant to preclinical and clinical studies of HSV-1 oncolysis.

Monotherapy of HCC with sorafenib reduces the risk of death during year 1 by 31 % and prolonged median survival and the time to progression by nearly 3 months (reviewed in Llovet et al., N Engl J Med (2008); 359: 378-390). Sorafenib is a standard of care treatment for HCC and whilst it is useful in treating HCC and prolonging patient survival a problem still exists as to how to improve the effectiveness of sorafenib on either time to progression or overall survival.

It is now also clear that drug resistance almost invariably develops in cancer patients treated with sorafenib (e.g. see Tang et al., Development of a resistance-like phenotype to sorafenib by human hepatocellular carcinoma cells is reversible and can be delayed by metronomic UFT chemotherapy. Neoplasia 2010 Nov; 12(1 1):928-40).

Doxorubicin and Sorafenib are cytostatic agents (Eichholtz-Wirth H., Br J Cancer 1980 Jun;41 (6):886-91 ; Rixe and Fojo., Clin Cancer Res December 15, 2007 13; 7280) meaning that they stop cell growth and division. Given that oncolytic viruses normally target growing and dividing cells, the cytostatic effect of doxorubicin and sorafenib would appear to be incompatible with the mechanism of action of an oncolytic virus.

WO2007/026146A1 describes the use of HSV, including oncolytic HSV of strain 17, in the treatment of a cancerous condition in a patient wherein the HSV is administered to the patient at a location outside of the cancerous condition that is to be treated.

US 2010/272691A1 describes herpes simplex virus comprising an N-terminally truncated glycoprotein D linked to a target agent.

### Summary of the Invention

In one aspect of the present invention HSV1716 for use in a method of treating hepatocellular carcinoma is provided according to the appended claims.

In some embodiments the use for the treatment further comprises administration of a chemotherapeutic agent, preferably doxorubicin and/or a kinase inhibitor, preferably sorafenib, and/or an anti-angiogenic, preferably bevacizumab and/or an EGFR inhibitor, preferably one or more of erlotinib, cetuximab or getifinib. In some embodiments the use, treatment or method of treatment further comprises simultaneous or sequential intra-arterial or intravenous administration of doxorubicin and/or oral administration of sorafenib. Administration of one or more, or each, of the oncolytic herpes simplex virus and doxorubicin may be to the hepatic artery or hepatic portal vein.

The oncolytic herpes simplex virus is HSV1716 (ECACC Accession No. V92012803).

Accordingly, HSV1716 for use in a method of treating hepatocellular carcinoma in a human patient is provided, the method comprising intravenous or intra-arterial administration of HSV1716 to the hepatic artery. In some embodiments the method further comprises simultaneous or sequential intra-arterial administration of doxorubicin to the hepatic artery or oral administration of sorafenib.

In another aspect of the present invention a pharmaceutical composition or medicament comprising HSV1716 and a chemotherapeutic agent is provided, wherein the chemotherapeutic agent is doxorubicin or sorafenib.

In another aspect of the present invention a kit comprising a predetermined amount of HSV1716 and a predetermined amount of chemotherapeutic agent is provided, wherein the chemotherapeutic agent is doxorubicin or sorafenib. The kit may be provided together with instructions for the administration of the oncolytic herpes simplex virus, doxorubicin, and/or sorafenib sequentially or simultaneously in order to provide a treatment for primary liver cancer.

In another aspect of the present invention products containing therapeutically effective amounts of:
(i) HSV1716, and
(ii) Doxorubicin and/or Sorafenib
for simultaneous or sequential use in a method of medical treatment, preferably treatment of primary liver cancer, are provided. The products may be pharmaceutically acceptable formulations and may optionally be formulated as a combined preparation for coadministration.

### Description of Preferred Embodiments

### Oncolytic Herpes Simplex Virus

An oncolytic virus is a virus that will lyse cancer cells (oncolysis), preferably in a selective manner. Viruses that selectively replicate in dividing cells over non-dividing cells are often oncolytic. Oncolytic viruses are well known in the art and are reviewed in Molecular Therapy Vol.18 No.2 Feb 2010 pg 233-234.

The oncolytic herpes simplex virus is preferably a replication-competent virus, being replication-competent at least in the target tumor cells.

The herpes simplex virus (HSV) genome comprises two covalently linked segments, designated long (L) and short (S). Each segment contains a unique sequence flanked by a pair of inverted terminal repeat sequences. The long repeat (RL or R_{L}) and the short repeat (RS or Rs) are distinct.

The HSV ICP34.5 (also called y34.5) gene, which has been extensively studied, has been sequenced in HSV-1 strains F and syn17+ and in HSV-2 strain HG52. One copy of the ICP34.5 gene is located within each of the RL repeat regions. Mutants inactivating one or both copies of the ICP34.5 gene are known to lack neurovirulence, i.e. be avirulent/ non-neurovirulent (non-neurovirulence is defined by the ability to introduce a high titre of virus (approx 10⁶ plaque forming units (pfu)) to an animal or patient without causing a lethal encephalitis such that the LD₅₀ in animals, e.g. mice, or human patients is in the approximate range of ≥10⁶ pfu), and be oncolytic.

Oncolytic HSV include HSV in which one or both of the γ34.5 (also called ICP34.5) genes are modified (e.g. by mutation which may
be a deletion, insertion, addition or substitution) such that the respective gene is incapable of expressing, e.g. encoding, a functional ICP34.5 protein. In HSV according to the invention both copies of the γ34.5 gene are modified such that the modified HSV is not capable of expressing, e.g. producing, a functional ICP34.5 protein.

Accordingly, in preferred embodiments the oncolytic herpes simplex virus is an ICP34.5 null mutant where all copies of the ICP34.5 gene present in the herpes simplex virus genome (two copies are normally present) are disrupted such that the herpes simplex virus is incapable of producing a functional ICP34.5 gene product.

Oncolytic herpes simplex virus may be derived from any HSV including any laboratory strain or clinical isolate (non-laboratory strain) of HSV.

A number of oncolytic herpes simplex viruses are known in the art. Examples include HSV1716, R3616 (e.g. see Chou & Roizman, Proc. Natl. Acad. Sci. Vol.89, pp.3266-3270, April 1992), G207 (Toda et al, Human Gene Therapy 9:2177-2185, October 10, 1995), NV1020 (Geevarghese et al, Human Gene Therapy 2010 Sep; 21 (9): 1 119-28), RE6 (Thompson et al, Virology 131 , 171-179 (1983)), and Oncovex™ (Simpson et al, Cancer Res 2006; 66:(9) 4835-4842 May 1 , 2006; Liu et al, Gene Therapy (2003): 10, 292-303).

In the present invention the herpes simplex virus is HSV-1 strain 17 mutant 1716 (HSV1716). HSV 1716 is an oncolytic, non-neurovirulent HSV and is described in EP 0571410, WO 92/13943, Brown et al (Journal of General Virology (1994), 75, 2367-2377) and MacLean et al (Journal of General Virology (1991), 72, 631-639). HSV 1716 has been deposited on 28 January 1992 at the European Collection of Animal Cell Cultures, Vaccine Research and Production Laboratories, Public Health Laboratory Services, Porton Down, Salisbury, Wiltshire, SP4 OJG, United Kingdom under accession number V92012803 in accordance with the provisions of the Budapest Treaty on the International Recognition of the Deposit of Microorganisms for the Purposes of Patent Procedure (herein referred to as the 'Budapest Treaty').

Oncolytic viruses may be formulated as medicaments and pharmaceutical compositions for clinical use and in such formulations may be combined with a pharmaceutically acceptable carrier, diluent or adjuvant. The composition may be formulated for intravenous or, intra-arterial routes of administration, which preferably include injection. Suitable formulations may comprise the virus in a sterile or isotonic medium. Medicaments and pharmaceutical compositions may be formulated in fluid (including gel) or solid (e.g. tablet) form. Fluid formulations may be formulated for administration by injection or via catheter to a selected region of the human or animal body. Medicaments and pharmaceutical compositions may be formulated in combination with one or more other active agents, e.g. a chemotherapeutic agent such as doxorubicin or sorafenib and/or an embolization agent and/or a contrast agent (e.g. Lipiodol).

Administration is preferably in a "therapeutically effective amount", this being sufficient to show benefit to the individual. The actual amount administered, and rate and time-course of administration, will depend on the nature and severity of the disease being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

Targeting therapies may be used to deliver the oncolytic virus to certain types of cell, e.g. by the use of targeting systems such as antibody or cell specific ligands. Targeting may be desirable for a variety of reasons; for example if the virus is unacceptably toxic in high dose, or if it would otherwise require too high a dosage, or if it would not otherwise be able to enter the target cells.

HSV capable of targeting cells and tissues are described in (PCT/GB2003/000603; WO 03/068809), hereby incorporated in its entirety by reference.

An oncolytic virus may be administered alone or in combination with other treatments, either simultaneously or sequentially dependent upon the condition to be treated. Such other treatments may include chemotherapy (including either systemic treatment with a chemotherapeutic agent or targeted therapy using small molecule or biological molecule (e.g. antibody) based agents that target key pathways in tumor development, maintenance or progression) or radiotherapy provided to the subject as a standard of care for treatment of the cancer.

Oncolytic herpes simplex virus may be administered in any therapeutically effective dosage amount. The inventors have identified that low dose of virus (∼<10⁶ pfu) is as effective in treating primary liver cancer as higher doses (∼>10⁷ pfu). In some embodiments a low dose of oncolytic herpes simplex virus is preferred, e.g. less than 1x10⁶ pfu, less than 1x10⁵ pfu or less than 1x10⁴ pfu.

### Cancer

A cancer may be any unwanted cell proliferation (or any disease manifesting itself by unwanted cell proliferation), neoplasm or tumor or increased risk of or predisposition to the unwanted cell proliferation, neoplasm or tumor. The cancer may be benign or malignant. A neoplasm or tumor may be any abnormal growth or proliferation of cells and may be located in any tissue.

The present invention is concerned with the treatment of primary liver cancer, i.e. a cancer originating in the liver. Examples of primary liver cancer include hepatocellular carcinoma (also called hepatoma), advanced hepatocellular carcinoma, biliary tree cancer, cholangiocarcinoma (bile duct cancer) and gallbladder cancer.

In some embodiments the primary liver cancer is an unresectable liver cancer, e.g. unresectable hepatocellular carcinoma. Determination of whether or not a primary liver cancer is resectable is within the skill and knowledge of a medical practitioner or surgeon working in the field of liver cancer.

### Subjects

The subject to be treated may be any animal or human. The subject is preferably mammalian, more preferably human. The subject may be a non-human mammal, but is more preferably human. The subject may be male or female. The subject may be a patient. A subject may have been diagnosed with a primary liver cancer, or be suspected of having a primary liver cancer prior to diagnosis.

### Chemotherapy

Chemotherapy refers to treatment of a tumor with a drug. For example, the drug may be a chemical entity, e.g. small molecule pharmaceutical, antibiotic, DNA intercalator, protein inhibitor (e.g. kinase inhibitor) or a biological agent, e.g. antibody, antibody fragment, nucleic acid or peptide aptamer, nucleic acid (e.g. DNA, RNA), peptide, polypeptide, or protein. The drug may be formulated as a pharmaceutical composition or medicament. The formulation may comprise one or more drugs (e.g. one or more active agents) together with one or more pharmaceutically acceptable diluents, excipients or carriers.

A treatment may involve administration of more than one drug. A drug may be administered alone or in combination with other treatments, either simultaneously or sequentially dependent upon the condition to be treated. For example, the chemotherapy may be a co-therapy involving administration of two drugs/agents, one or more of which may be intended to treat the tumor. In the present invention an oncolytic herpes simplex virus and chemotherapeutic may be administered simultaneously, separately, or sequentially which may allow the two agents be present in the tumor requiring treatment at the same time and thereby provide a combined therapeutic effect, which may be additive or synergistic.

The chemotherapy may be administered by one or more routes of administration, e.g. parenteral, intra-arterial injection or infusion, intravenous injection or infusion, intraperitoneal, intratumoral or oral. Administration is preferably in a "therapeutically effective amount", this being sufficient to show benefit to the individual. The actual amount administered, and rate and time-course of administration, will depend on the nature and severity of the disease being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

The chemotherapy may be administered according to a treatment regime. The treatment regime may be a pre-determined timetable, plan, scheme or schedule of chemotherapy administration which may be prepared by a physician or medical practitioner and may be tailored to suit the patient requiring treatment.

The treatment regime may indicate one or more of: the type of chemotherapy to administer to the patient; the dose of each drug; the time interval between administrations; the length of each treatment; the number and nature of any treatment holidays, if any etc. For a co-therapy a single treatment regime may be provided which indicates how each drug/agent is to be administered.

### Chemotherapeutic agents

Chemotherapeutic agents known for use in treating primary liver cancer include 5-Fluorouracil, floxuridine (FUDR), leucovorin, oxaliplatin, irinotecan, doxorubicin and sorafenib.

Some chemotherapeutic agents may be administered by hepatic arterial infusion (HAI) which allows for delivery directly to the liver.

Doxorubicin ((7S,9S)-7-[(2R,4S,5S,6S)-4-amino-5-hydroxy-6-methyloxan-2-yl]oxy-6,9,11-trihydroxy-9-(2-hydroxyacetyl)-4-methoxy-8,10-dihydro-7H-tetracene-5,12-dione), marketed under the trade name Adriamycin™ (amongst others). Doxorubicin is an anthracycline antibiotic and DNA intercalator.

Doxorubicin (adriamycin, DOX) is routinely used as a single agent in advanced HCC and is frequently an active chemotherapeutic in TACE. However, the objective response rate of HCC to doxorubicin is only 10-20% and there is no reported survival benefit (Yeo et al., J. Natl. Cancer Inst. 2005; 97: 1532-1538, Furuse, Biologics 2008; 2: 779-788). Doxorubicin interacts with DNA by intercalation and inhibits topoisomerase II. Doxorubicin stabilizes the DNA-topoisomerase II complex after the DNA chain cleavage required for the relaxation of DNA supercoils, preventing the DNA double helix from being resealed. Accumulated DNA damage after doxorubicin exposure kills cancer cells most likely via apoptosis although its precise mode of action is not fully understood.

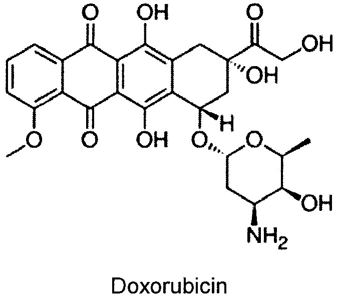

Typical dosages of doxorubicin for treatment of human patients may be in the range 40-60mg/m² (iv).

Sorafenib (4-[4-[[4-chloro-3-(trifluoromethyl)phenyl]carbamoylamino]phenoxy]-*N*-methylpyridine-2-carboxamide), marketed under the trade name Nexavar™, is a tyrosine kinase and Raf kinase inhibitor and targets the Raf/Mek/Erk (MAP Kinase) pathway.

Sorafenib (Bay43-9006) is a small molecule tyrosine kinase inhibitor principally targeting VEGFR2, which is thought to mediate almost all of the known cellular responses to VEGF and therefore inhibiting angiogenesis. Sorafenib also inhibits PDGFR, Raf and c-Kit and inhibition of the Raf/MEK/ERK signalling axis abrogated tumour growth (Wilhelm et al., Mol Cancer Ther (2008); 7: 3129-3140). Sorafenib was co-developed and co-marketed by Bayer and Onyx as Nexavar and was approved by the FDA/EMA in 2005/2006 for use in the treatment of advanced RCC, and by EMA/FDA in 2007 for advanced HCC. Monotherapy of HCC with sorafenib reduces the risk of death during year 1 by 31% and prolonged median survival and the time to progression by nearly 3 months (reviewed in Llovet et al., N Engl J Med (2008); 359: 378-390).

Sorafenib has received EU marketing authorisation and FDA approval for the treatment of HCC.

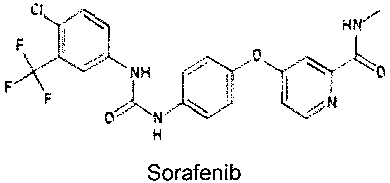

Typical dosages of sorafenib for treatment of human patients may be about 200mg or 400mg per day (oral) or about 5-20mg/kg (iv).

In this specification reference to doxorubicin and sorafenib includes their salts (e.g. hydrochloride or tosylate salts), hydrates, prodrug formulations and other pharmaceutically acceptable formulations that provide the active doxorubicin or sorafenib agent when administered to the human or mammalian body.

### Forms of Chemotherapeutic Agent

The active compound of a given chemotherapeutic agent may be provided in the form of a corresponding salt, solvate, or prodrug. In this specification reference to the chemotherapeutic agent includes reference to such forms.

### Salts

It may be convenient or desirable to prepare, purify, and/or handle a corresponding salt of the active compound, for example, a pharmaceutically-acceptable salt. Examples of pharmaceutically acceptable salts are discussed in Berge et al., 1977, "Pharmaceutically Acceptable Salts," J. Pharm. Sci., Vol. 66, pp. 1-19.

For example, if the compound is anionic, or has a functional group which may be anionic (e.g., -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al⁺³. Examples of suitable organic cations include, but are not limited to, ammonium ion (i.e., NH₄⁺) and substituted ammonium ions (e.g., NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group which may be cationic (e.g., -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose.

Unless otherwise specified, a reference to a particular compound also include salt forms thereof.

### Solvates

It may be convenient or desirable to prepare, purify, and/or handle a corresponding solvate of the active compound. The term "solvate" is used herein in the conventional sense to refer to a complex of solute (e.g., active compound, salt of active compound) and solvent. If the solvent is water, the solvate may be conveniently referred to as a hydrate, for example, a mono-hydrate, a di-hydrate, a tri-hydrate, etc.

Unless otherwise specified, a reference to a particular compound also include solvate forms thereof.

### Prodrugs

It may be convenient or desirable to prepare, purify, and/or handle the active compound in the form of a prodrug. The term "prodrug," as used herein, pertains to a compound which, when metabolised (e.g., in vivo), yields the desired active compound. Typically, the prodrug is inactive, or less active than the active compound, but may provide advantageous handling, administration, or metabolic properties.

Unless otherwise specified, a reference to a particular compound also include prodrugs thereof.

For example, some prodrugs are esters of the active compound (e.g., a physiologically acceptable metabolically labile ester). During metabolism, the ester group (-C(=O)OR) is cleaved to yield the active drug. Such esters may be formed by esterification, for example, of any of the carboxylic acid groups (-C(=O)OH) in the parent compound, with, where appropriate, prior protection of any other reactive groups present in the parent compound, followed by deprotection if required.

Examples of such metabolically labile esters include those of the formula -C(=O)OR wherein R is: C₁₋₇alkyl (e.g., -Me, -Et, -nPr, -iPr, -nBu, -sBu, -iBu, -tBu); C₁₋₇aminoalkyl (e.g., aminoethyl; 2-(N,N-diethylamino)ethyl; 2-(4-morpholino)ethyl); and acyloxy-C₁₋₇alkyl (e.g., acyloxymethyl; acyloxyethyl; pivaloyloxymethyl; acetoxymethyl; 1-acetoxyethyl; 1-(1-methoxy-1-methyl)ethyl-carbonxyloxyethyl; 1-(benzoyloxy)ethyl; isopropoxycarbonyloxymethyl; 1-isopropoxy-carbonyloxyethyl; cyclohexyl-carbonyloxymethyl; 1-cyclohexyl-carbonyloxyethyl; cyclohexyloxy-carbonyloxymethyl; 1-cyclohexyloxycarbonyloxyethyl; (4-tetrahydropyranyloxy) carbonyloxymethyl; 1-(4-tetrahydropyranyloxy)carbonyloxyethyl; (4-tetrahydropyranyl)carbonyloxymethyl; and 1-(4-tetrahydropyranyl)carbonyloxyethyl).

Also, some prodrugs are activated enzymatically to yield the active compound, or a compound which, upon further chemical reaction, yields the active compound (for example, as in ADEPT, GDEPT, LIDEPT, etc.). For example, the prodrug may be a sugar derivative or other glycoside conjugate, or may be an amino acid ester derivative.

### Other Chemotherapeutic Agents

In addition to treating a cancer by using an oncolytic herpes simplex virus optionally in combination with doxorubicin or sorafenib, subjects being treated may also receive treatment with other chemotherapeutic agents. For example, other chemotherapeutic agents may be selected from:
(i) alkylating agents such as cisplatin, carboplatin, mechlorethamine, cyclophosphamide, chlorambucil, ifosfamide;
(ii) purine or pyrimidine anti-metabolites such as azathiopurine or mercaptopurine;
(iii) alkaloids and terpenoids, such as vinca alkaloids (e.g. vincristine, vinblastine, vinorelbine, vindesine), podophyllotoxin, etoposide, teniposide, taxanes such as paclitaxel (Taxol™), docetaxel;
(iv) topoisomerase inhibitors such as the type I topoisomerase inhibitors camptothecins irinotecan and topotecan, or the type II topoisomerase inhibitors amsacrine, etoposide, etoposide phosphate, teniposide;
(v) antitumor antibiotics (e.g. anthracyline antibiotics) such as dactinomycin, doxorubicin (Adriamycin™), epirubicin, bleomycin, rapamycin;
(vi) antibody based agents, such as anti-VEGF, anti-TNFa, anti-IL-2, antiGpIIb/IIIa, anti-CD-52, anti-CD20, anti-RSV, anti-HER2/neu(erbB2), anti-TNF receptor, anti-EGFR antibodies, monoclonal antibodies or antibody fragments, examples include: cetuximab, panitumumab, infliximab, basiliximab, bevacizumab (Avastin®), abciximab, daclizumab, gemtuzumab, alemtuzumab, rituximab (Mabthera®), palivizumab, trastuzumab, etanercept, adalimumab, nimotuzumab,
(vii) EGFR inihibitors such as erlotinib, cetuximab and gefitinib,
(viii) anti-angiogenic agents such as bevacizumab (Avastin®).

### Simultaneous or Sequential Administration

Compositions may be administered alone or in combination with other treatments, either simultaneously or sequentially dependent upon the condition to be treated.

In this specification an oncolytic herpes simplex virus and chemotherapeutic agent may be administered simultaneously or sequentially.

Simultaneous administration refers to administration of the oncolytic herpes simplex virus and chemotherapeutic agent together, for example as a pharmaceutical composition containing both agents, or immediately after each other via the same route of administration, e.g. to the same artery, vein or other blood vessel.

Sequential administration refers to administration of one of the oncolytic herpes simplex virus or chemotherapeutic agent followed after a given time interval by separate administration of the other agent. It is not required that the two agents are administered by the same route, although in some embodiments this preferred. For example, in some embodiments one of the oncolytic herpes simplex virus or chemotherapeutic agent is administered by intra-arterial or intravenous administration followed, after a predetermined time interval, by administration of the other agent to the same artery or vein, preferably to the same site, e.g. by re-use of a catheter or tube inserted in the patient's artery or vein. The predetermined time interval may be any time interval.

Whilst simultaneous or sequential administration is intended such that both the oncolytic herpes simplex virus and chemotherapeutic agent are delivered to the same tumor tissue to effect treatment it is not essential for both agents to be present in the tumor tissue in active form at the same time.

However, in some embodiments of sequential administration the time interval is selected such that the oncolytic herpes simplex virus and chemotherapeutic agent are expected to be present in the tumor tissue in active form at the same time, thereby allowing for a combined, additive or synergistic effect of the two agents in treating the tumor. In such embodiments the time interval selected may be any one of 5 minutes or less, 10 minutes or less, 15 minutes or less, 20 minutes or less, 25 minutes or less, 30 minutes or less, 45 minutes or less, 60 minutes or less, 90 minutes or less, 120 minutes or less, 180 minutes or less, 240 minutes or less, 300 minutes or less, 360 minutes or less, or 720 minutes or less, or 1 day or less, or 2 days or less.

### Intravenous administration

Infusion of active agents (e.g. oncolytic herpes simplex virus and/or chemotherapeutic agent(s)) to a vein is a well-developed and well-known means of delivering therapeutic agents to the body in a systemic manner.

### Intra-arterial administration

Infusion of active agents (e.g. oncolytic herpes simplex virus and/or chemotherapeutic agent(s)) to the hepatic artery has been developed as a means of directly delivering therapeutic and imaging/diagnostic agents to the liver.

At its simplest, a catheter is inserted to the patient's vasculature and is manipulated to gain access to the hepatic artery. Active agents may then be administered to the blood flowing directly to the liver. Where more than one agent is to be delivered, the agents may be administered together, i.e. in combination, or separately but over a period of time that allows for both agents to be present in the liver at the same time and thereby allow for them to exert a combined or synergistic effect.

In practice, intra-arterial infusion is preferred, although in principle a similar infusion technique could be applied to deliver agents to the liver via the hepatic portal vein.

One technique developed for intra-arterial infusion suitable in the context of treatment of primary liver cancer is trans-arterial chemoembolization (TACE).

TACE is normally performed by an interventional radiologist and involves accessing the hepatic artery with a catheter, which is possible by puncturing the common femoral artery in the right groin and passing a catheter through the abdominal aorta, through the celiac trunk and common hepatic artery, into the proper hepatic artery.

An arteriogram is performed to identify the branches of the hepatic artery supplying the tumor(s). Smaller catheters may then be threaded into these branches (so-called superselective positioning). This allows precision delivery of the active agents to the tumor tissue.

Once the catheter is in position, doses of the active agent (e.g. oncolytic herpes simplex virus, and/or chemotherapeutic agent and/or embolisation agent and/or contrast agent) are injected through the catheter. The total dose may be given to a single vessel, or if there are several tumor foci may be divided among several vessels supplying the tumors.

Because most tumors are supplied by the hepatic artery, arterial embolization interrupts the blood supply to the tumor and delays tumor growth. The focused nature of the administration of active agents enables delivery of a high therapeutic dose to the tissue requiring treatment whilst reducing systemic exposure and therefore toxicity. Embolization of the vessel assists this process in that the active agent(s) is not washed out from the tumor bed and the supply of nutrients to the tumor is decreased thereby promoting tumor necrosis.

TACE is widely used as a palliative treatment for surgically unresectable primary or metastatic HCC tumors.

### Intratumoral administration

Intratumoral administration of active agents is a well-developed and well-known means of directly delivering therapeutic agents to a tumor. Delivery is typically by injection, which in some instances may require use of stereotactic injection equipement.

### Other Treatments

Administration of an oncolytic herpes simplex virus and optionally chemotherapeutic agent in accordance with the present invention may be combined with other medical procedures such surgical resection of the tumor or embolization (e.g. chemical embolization) of the tumor.

### Dosage regime

The inventors have identified that multiple doses of oncolytic herpes simplex virus provide greater efficacy in reducing tumor volume and tumor remission. Accordingly, multiple doses of the oncolytic virus may be provided. One or more, or each, of the doses may be accompanied by simultaneous or sequential administration of a chemotherapeutic agent.

Multiple doses may be separated by a predetermined time interval, which may be selected to be one of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days, or 1, 2, 3, 4, 5, or 6 months.

By way of example, doses may be given once every 7, 14, 21 or 28 days (plus or minus 3, 2, or 1 days). The dose of oncolytic herpes simplex virus given at each dosing point may be the same, but this is not essential. For example, it may be appropriate to give a higher priming dose at the first, second and/or third dosing points.

### Kits

In some aspects of the present invention a kit of parts is provided. In some embodiments the kit may have at least one container having a predetermined quantity of oncolytic herpes simplex virus, e.g. predetermined viral dose or number/quantity/concentration of viral particles. The oncolytic herpes simplex virus may be formulated so as to be suitable for injection or infusion to the blood. In some embodiments the kit may further comprise at least one container having a predetermined quantity of chemotherapeutic agent, e.g. doxorubicin or sorafenib. The chemotherapeutic agent may also be formulated so as to be suitable for injection or infusion to the blood. In some embodiments a container having a mixture of a predetermined quantity of oncolytic herpes simplex virus and predetermined quantity of chemotherapeutic agent is provided, which may optionally be formulated so as to be suitable for injection or infusion to the blood.

In some embodiments the kit may also contain apparatus suitable to administer one or more doses of the oncolytic herpes simplex virus and/or chemotherapeutic agent. Such apparatus may include one or more of a catheter and/or needle and/or syringe, such apparatus preferably being provided in sterile form.

The kit may further comprise instructions for the administration of a therapeutically effective dose of the oncolytic herpes simplex virus and/or chemotherapeutic agent. The instructions may include instructions for administration to the hepatic artery or hepatic portal vein, or for oral administration.

The invention includes the combination of the aspects and preferred features described except where such a combination is clearly impermissible or expressly avoided.

The section headings used herein are for organizational purposes only and are not to be construed as limiting the subject matter described.

Aspects and embodiments of the present invention will now be illustrated, by way of example, with reference to the accompanying figures. Further aspects and embodiments will be apparent to those skilled in the art.

### Brief Description of the Figures

Embodiments and experiments illustrating the principles of the invention will now be discussed with reference to the accompanying figures in which:
**Figure 1****.** Graph showing yields from Huh-7 cells 72 hours after infection with different multiplicities of infection of HSV1716 or HSV-1 17+.
**Figure 2****.** Graph showing yields from Huh-7 cells at 24, 48 and 72 hours after infection with HSV-1 17+ or HSV1716 at various multiplicities of infection.
**Figure 3****.** Graph showing replication of HSV-1 17+ and HSV1716 in HepG2-luc2 cells, showing virus titres at 48 and 72 hours after infection of HepG2-luc2 cells with HSV-1 17+ and HSV1716 at multiplicities of infection of 0.1 or 0.01.
**Figure 4****.** Graph showing HepG2-luc2 luciferase activity (luminosity) at 24 hours and 48 hours post infection with HSV-1 17+ and HSV1716 at multiplicities of infection of 1 or 0.1.

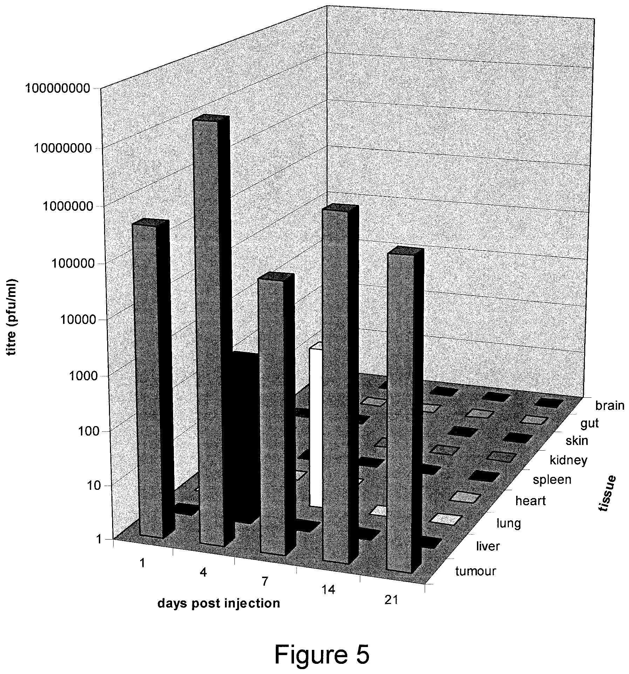

**Figure 5****.** Chart showing biodistribution by titre in nude mice bearing Huh-7 xenografts following intravenous injection of HSV1716.
**Figure** 6. Graph showing growth of HuH-7 tumor xenografts as far as time at which no virus controls were sacrificed. Mice were exposed to no virus, or HSV1716 at 1x10⁶ pfu or 1x10⁷ pfu, administered by tail vein injection on days 1 and 4.
**Figure 7****.** Graph showing growth of HuH-7 tumor xenografts as far as end of the experiment. Mice were exposed to no virus, or HSV1716 at 1x10⁶ pfu or 1x10⁷ pfu, administered by tail vein injection on days 1 and 4.
**Figure** 8. Graph showing survival of mice having HuH-7 xenografts following treatment with no virus, or HSV1716 at 1x10⁶ pfu or 1x10⁷ pfu, administered by tail vein injection on days 1 and 4.

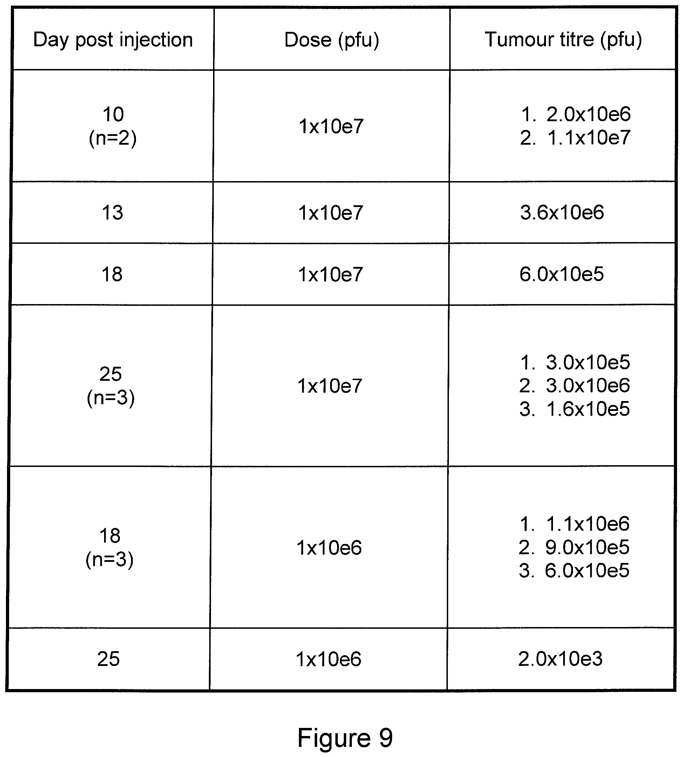

**Figure 9****.** Table showing titration of HSV1716 in HuH-7 tumor extracts.
**Figure 10****.** Graph showing survival of mice having HuH-7 xenografts following treatment with no virus, or HSV1716 at 1x10⁵ pfu, 1x10⁶ pfu or 1x10⁷ pfu, administered by tail vein injection on days 1, 14 and 29.
**Figure 11****.** Graph showing growth of HuH-7 tumor xenografts as far as end of the experiment. Mice were exposed to no virus, or HSV1716 at 1x10⁵ pfu, 1x10⁶ pfu or or 1x10⁷ pfu, administered by tail vein injection on days 1, 14 and 29.

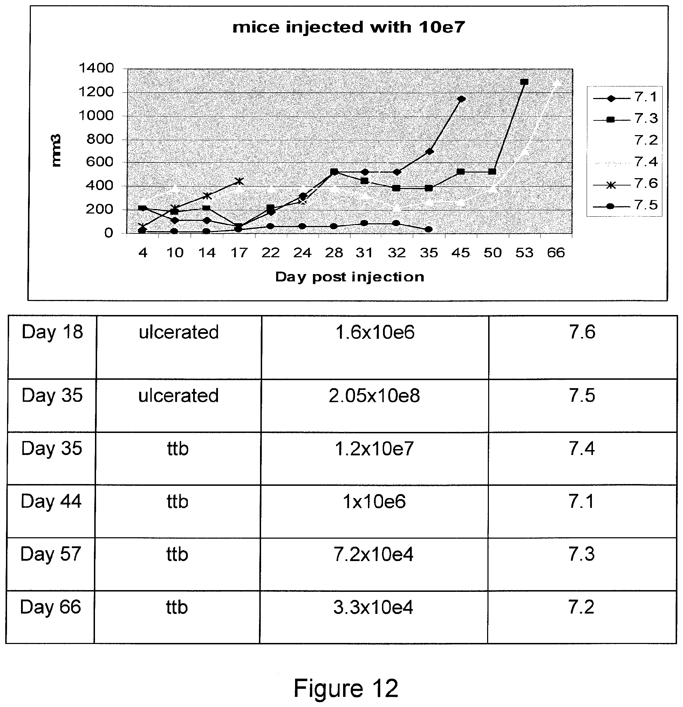

**Figure 12****.** Graph showing growth of tumor and table showing intratumoral viral titre in individual mice treated with 1x10⁷ pfu HSV1716 by tail vein injection on days 1, 14 and 29.

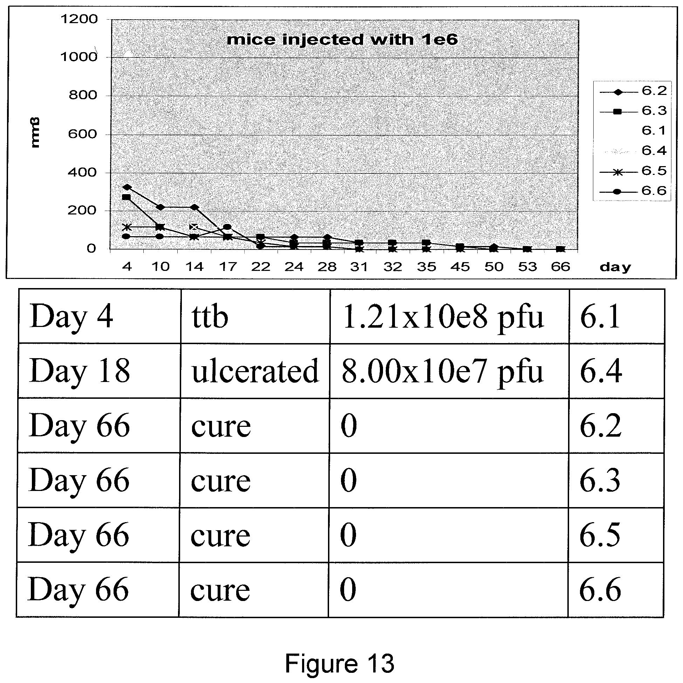

**Figure** 13. Graph showing growth of tumor and table showing intratumoral viral titre in individual mice treated with 1x10⁶ pfu HSV1716 by tail vein injection on days 1, 14 and 29.

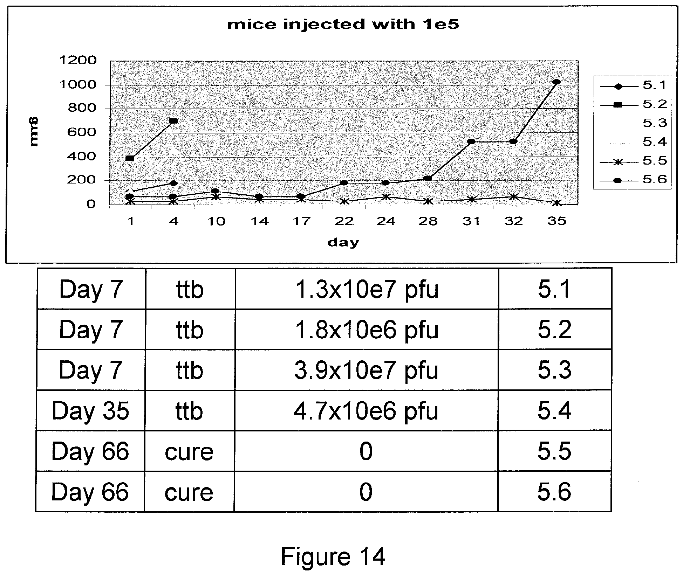

**Figure 14****.** Graph showing growth of tumor and table showing intratumoral viral titre in individual mice treated with 1x10⁵ pfu HSV1716 by tail vein injection on days 1, 14 and 29.
**Figure 15****.** Table and Graph showing infection of HuH-7 cells in tumor xenografts by HSV1716 after intravenous administration.
**Figure 16****.** Table showing infection of HuH-7 cells in tumor xenografts by HSV1716 after intravenous administration of varying doses of HSV1716.
**Figure 17****.** Chart showing HSV1716 localisation to HuH-7 mouse xenografts.
**Figure 18****.** Chart showing biodistribution by luciferase activity in nude mice bearing HuH-7 xenografts following intravenous injection of HSV1716luc.

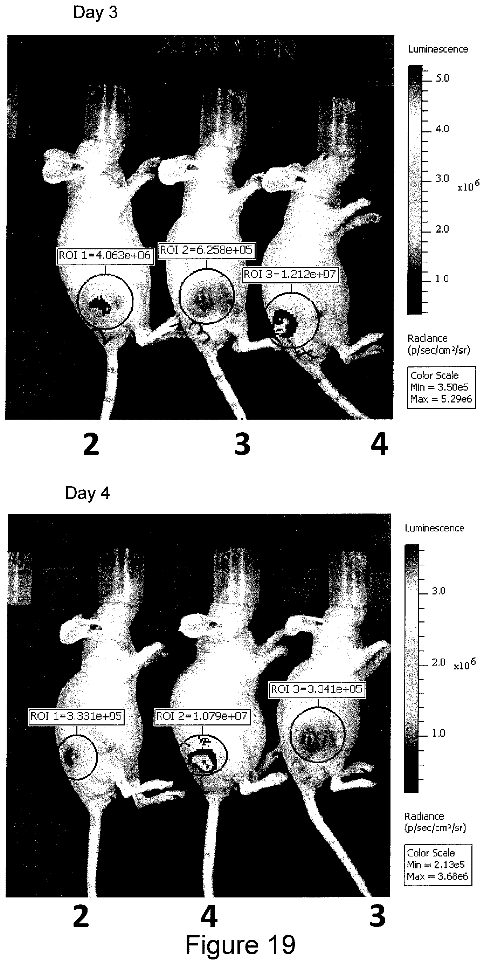

**Figure 19****.** Photographs showing luciferase expression in "dox 1" treated nude mice at day 3 and day 4 following intratumoral injection.

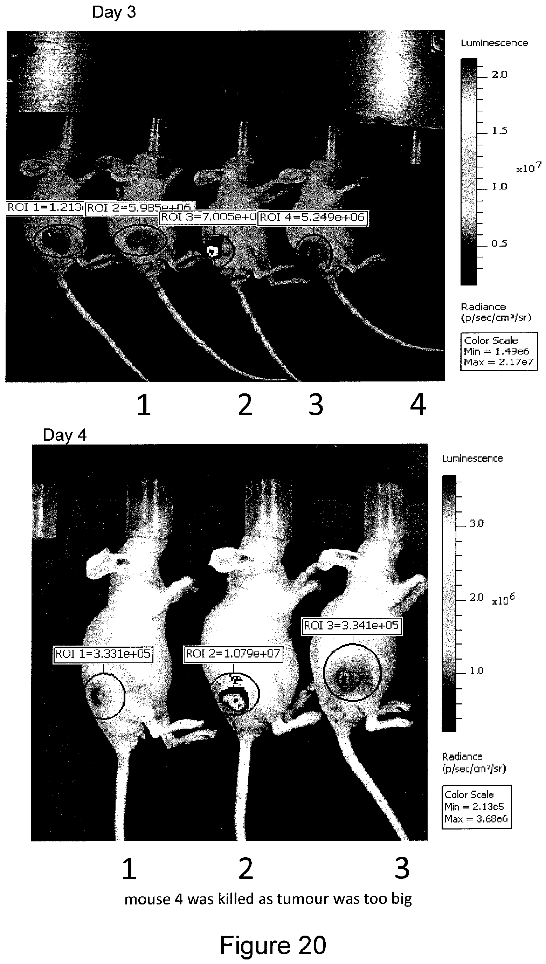

**Figure 20****.** Photographs showing luciferase expression in "dox 2" treated nude mice at day 3 and day 4 following intratumoral injection. NB by day 4 mouse 4 was killed because the tumor was too large.
**Figure 21****.** Charts showing light emissions of "dox 1" and "dox 2" treated mice at days 3, 4, 11 and 14 following intratumoral injection.

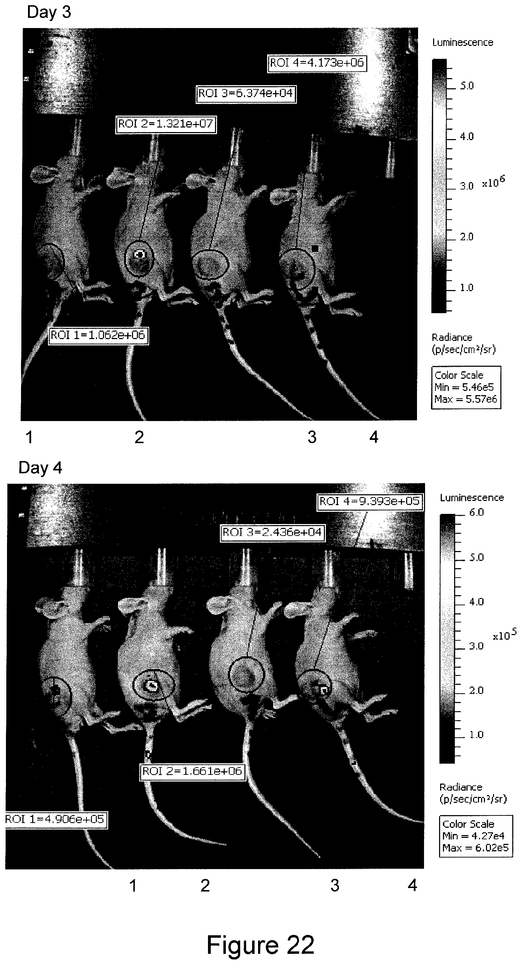

**Figure 22****.** Photographs showing luciferase expression in "dox 4" treated nude mice at day 3 and day 4 following administration.

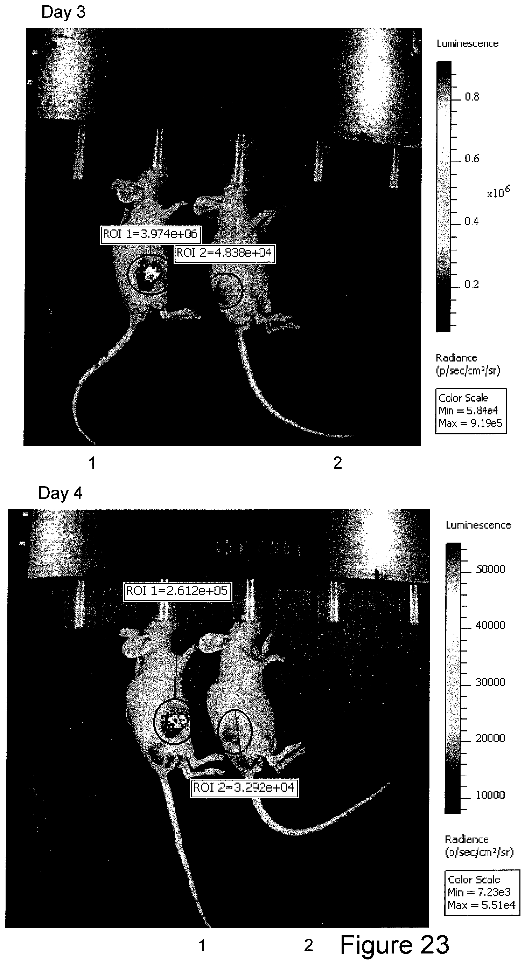

**Figure 23****.** Photographs showing luciferase expression in "dox 5" treated nude mice at day 3 and day 4 following administration.
**Figure 24****.** Charts showing light emissions of "dox 4" and "dox 5" treated mice at days 3, 4, 7 and 11 following administration.

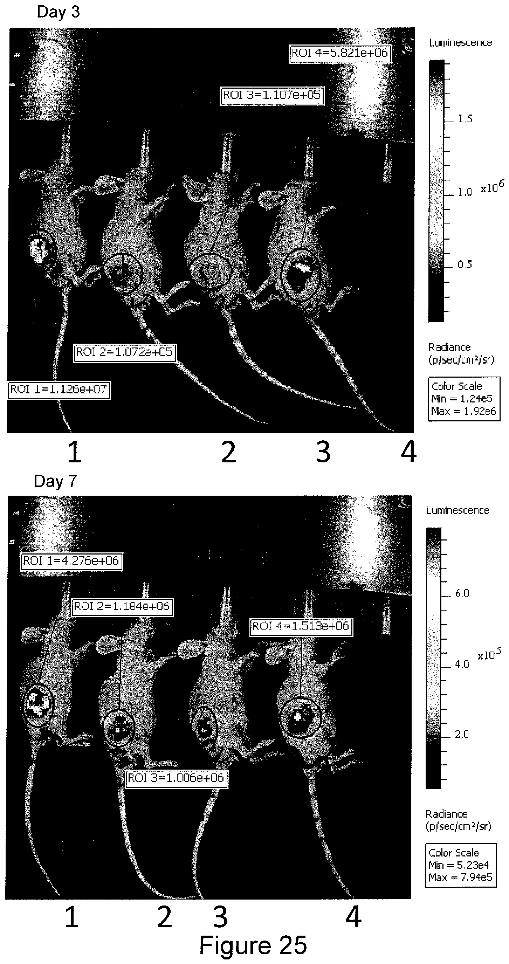

**Figure 25****.** Photographs showing luciferase expression in "dox 3" treated nude mice at day 3 and day 7 following administration.
**Figure 26****.** Chart showing light emissions of "dox 3" treated mice at days 3, 4 and 7 following administration.
**Figure 27****.** Chart showing comparison of viral titre in tumor extracts vs. survival. d1=dox 1, d2=dox 2, d3=dox 3, d4=dox 4, d5=dox 5, m1=mouse 1 etc.
**Figure 28****.** Graph showing progeny yields/input virus from HSV-1 17+ and HSV1716 infection of HuH7, HepG2-luc, SKOV3, SPC111, U87 and A431 cells.

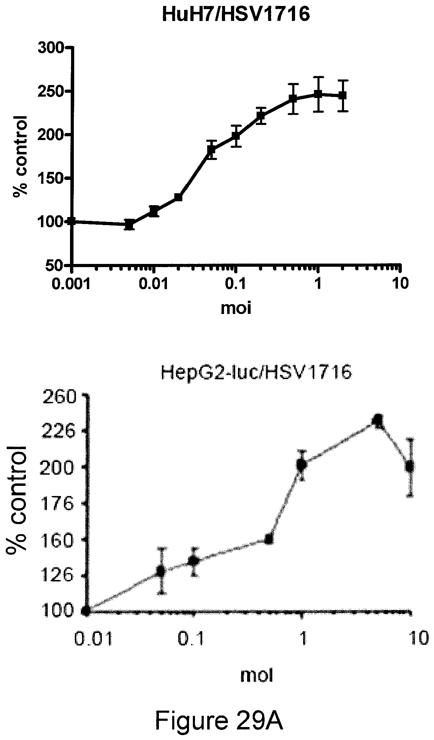

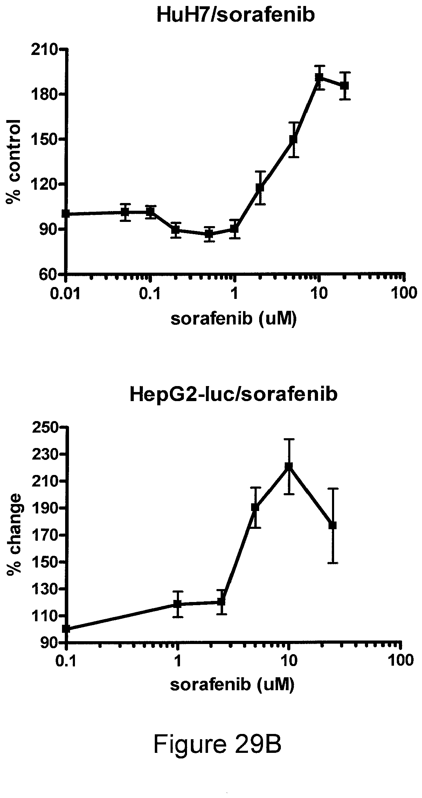

**Figure 29****.** Graphs showing dead cell protease leakage values expressed as % control (no virus or no drug). (a) HSV1716 was added at moi 2, 1, 0.5, 0.2, 0.1, 0.05, 0.02, 0.01 and 0.005, and (b) sorafenib was added to give final concentrations of 20, 10, 5, 2, 1, 0.5, 0.2, 0.1 and 0.05 µM to HuH7 and HepG2-luc cells in culture.
**Figure 30****.** Table showing moi/drug concentrations used for combination studies. Sorafenib and HSV1716 IC50 for HuH7 and HepG2-luc cells. *IC50s reported by Chang and Wang, Anti-Cancer Drugs 2013; 24: 251-259.

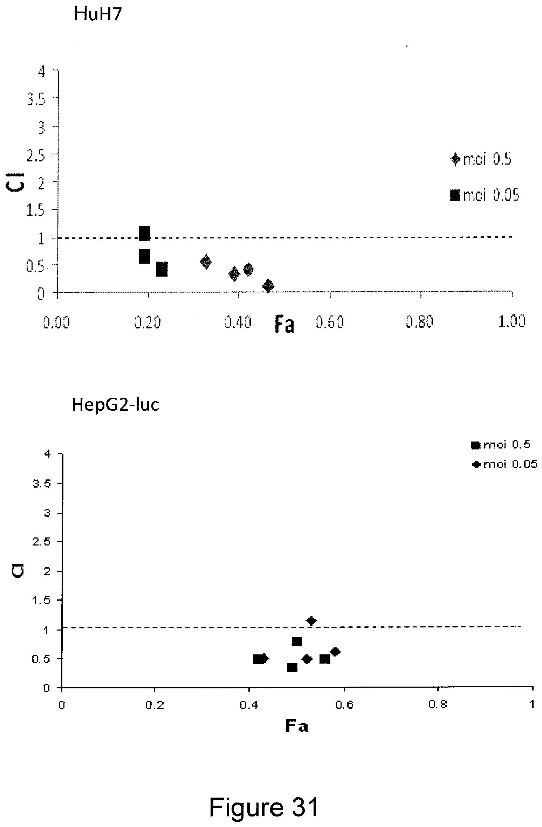

**Figure 31****.** FaCI plots for the effects of combined sorafenib and HSV1716 treatment on HuH7 and HepG2-luc cells. HSV1716 was added at moi 0.5 and 0.05 and sorafenib was added at 2.5, 1, 0.5, and 0.25 µM. The Combination Index (y-axis) was plotted against Fa (x-axis) and the line at CI=1 divides synergy (below) from antagonism (above). Most combinations of sorafenib with HSV1716 at both moi generate CI values of <1 and therefore the drug acts synergistically to enhance HuH7 and HepG2-luc cell death in vitro.
**Figure 32****.** Table showing summary of Chou-Talalay analysis of HSV1716 in combination with sorafenib in 2 human HCC cell lines. (+) = synergy at most moi and drug concentrations, (+/-) = mixed signals with some combinations synergistic and others antagonistic, - mostly antagonistic in combination.
**Figure 33****.** Graphs showing dead cell protease leakage values expressed as % control (no virus or no drug). (a) HSV1716 was added at moi 2, 1, 0.5, 0.2, 0.1, 0.05, 0.02, 0.01 and 0.005 and (b) doxorubicin was added to give final concentrations of 12.5, 2.5, 1.25, 0.25, 0.125, 0.025, 0.0125 and 0.001 uM µM to HuH7 and HepG2-luc cells in culture.
**Figure 34****.** Table showing moi/drug concentrations used for combination studies. Doxorubicin and HSV1716 IC50 for HuH7 and HepG2-luc cells. *IC50s reported by Chang and Wang, Anti-Cancer Drugs 2013; 24: 251-259.

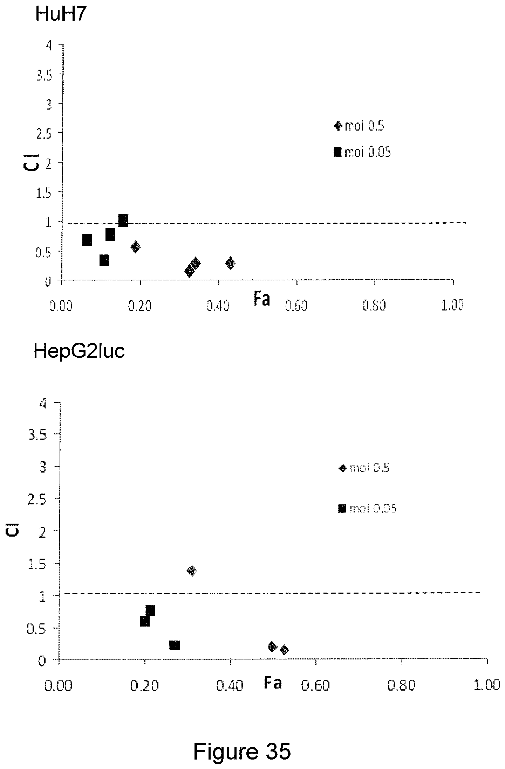

**Figure 35****.** FaCI plots for the effects of combined doxorubicin and HSV1716 treatment on HuH7 and HepG2-luc cells. HSV1716 was added at moi 0.5 and 0.05 and doxorubicin was added at 0.1, 0.05, 0.01 and 0.05 µM. The Combination Index (y-axis) was plotted against Fa (x-axis) and the line at CI=1 divides synergy (below) from antagonism (above). CI values > 4 will not appear on the plots. Most combinations of HSV1716 with doxorubicin in HuH7 and HepG2-luc acted synergistically to enhance cell death.

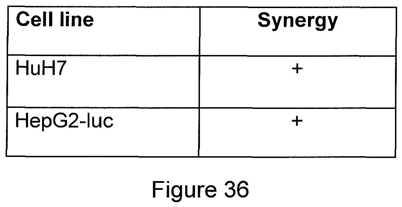

**Figure 36****.** Table showing summary of Chou-Talalay analysis of HSV1716 in combination with doxorubicin in 2 human HCC cell lines. (+) = synergy at most moi and drug concentrations, (+/-) = mixed signals with some combinations synergistic and others antagonistic, - mostly antagonistic in combination.
**Figure 37****.** Chart showing growth of HuH7 xenografts after no treatment (c), HSV1716 (v), sorafenib (s) and HSV1716/sorafenib (v+s) in HuH7 xenografts.
**Figure 38****.** Chart showing survival of nude mice with HuH7 xenografts after no treatment (c), HSV1716 (v), sorafenib (s) and HSV1716/sorafenib (v+s) in HuH7 xenografts.
**Figure 39****.** Chart showing effects of cell plating density on light output from HepG-luc cells. Mean +/- sd from eight determinations are shown.
**Figure 40****.** Chart showing HepG2-luc output 24 hours after infection with HSV-1 17+, 1716 or HSV1716ING4.
**Figure 41****.** Chart showing virus titres at 48 and 72 hrs after infection of HepG2-luc with 17+ or 1716 at mois of 0.1 or 0.01.
**Figure 42****.** IVIS images of 3 groups of mice having HepG2-luc xenografts during treatment with either no virus, HSV1716 administered intratumorally (IT) or HSV1716 administered intravenously.
**Figure 43****.** IVIS images of mice injected with 2x10⁴ PFU HSV1716 intratumourally. Graph insert shows loss of light from the xenograft regions of interest for the treated mice from >10⁷ radiance to the background level of c10⁵ radiance.
**Figure 44****.** IVIS images of mice injected with 2x10⁶ PFU HSV1716 intratumourally.
**Figure 45****.** IVIS images of no virus control mice at later time points when most treated mice have lost their luciferase signal.
**Figure 46****.** IVIS images treatment of control mice which developed HepG2-luc tumours with a single IT injection of 1x10⁶ pfu HSV1716. The graph insert shows loss of light from the xenograft regions of interest for the two mice from >10⁸ radiance to the background level of c10⁴ radiance.

### Detailed Description of the Invention

The details of one or more embodiments of the invention are set forth in the accompanying description below including specific details of the best mode contemplated by the inventors for carrying out the invention, by way of example.

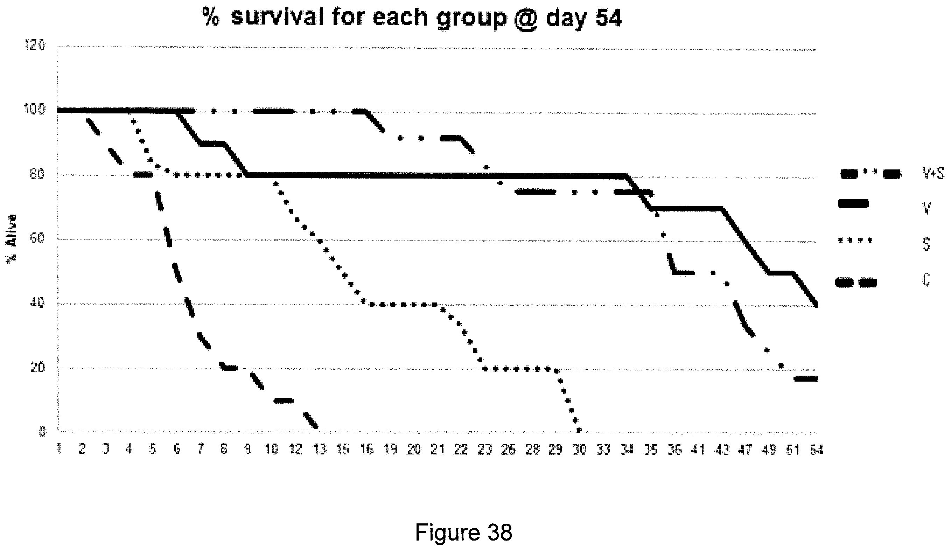

The inventors have shown HSV1716 to exhibit better replication kinetics in HCC cells compared to the parent wild type strain HSV-1 17+. This is an unusual and surprising finding because an attenuated HSV would normally be expected to exhibit a reduction in replication kinetics compared to its corresponding parent strain, as shown to be the case in the other tumor cell lines tested (ovarian, mesothelioma and high grade glioma). HCC tumors are normally slow growing which makes the result obtained even more surprising. Yet this finding indicates a marked potential efficacy of HSV1716 for the treatment of HCC. Furthermore, the combination of oncolytic virus and sorafenib is shown to extend the period in which no mice died from about 4 days with sorafenib or about 7 days with virus only to about 19 days with virus and sorafenib (Figure 38). This indicates an extension of the survival benefit that is provided by sorafenib alone.

### Examples

### Example 1 - In vitro infection of HCC lines with HSV1716

### Hepatocellular carcinoma cells

HuH-7 is a well-differentiated, hepatocyte-derived cellular carcinoma cell line that was originally taken from a liver tumor in a 57-year-old Japanese male (Vecchi et al., Hepatology 2010 Feb; 51(2):654-9). HuH-7 are epithelial-like tumorigenic cells and are fully permissive for HSV1716.

The HepG2 cell line (ATCC No. HB-8065) was derived from the liver tissue of a fifteen year old male with differentiated hepatocellular carcinoma. HepG2 are adherent, epithelial-like cells growing as monolayers and in small aggregates. HepG2 are fully permissive for HSV1716.

HepG2-*luc*2 (Caliper HT1080-*luc2)* is a luciferase expressing cell line stably transfected with the firefly luciferase gene (*luc*2)*.* The cell line was established by transducing lentivirus containing luciferase 2 gene under the control of human ubiquitin C promoter.

### In vitro HSV1716 replication in HuH7 and HepG2-luc cells

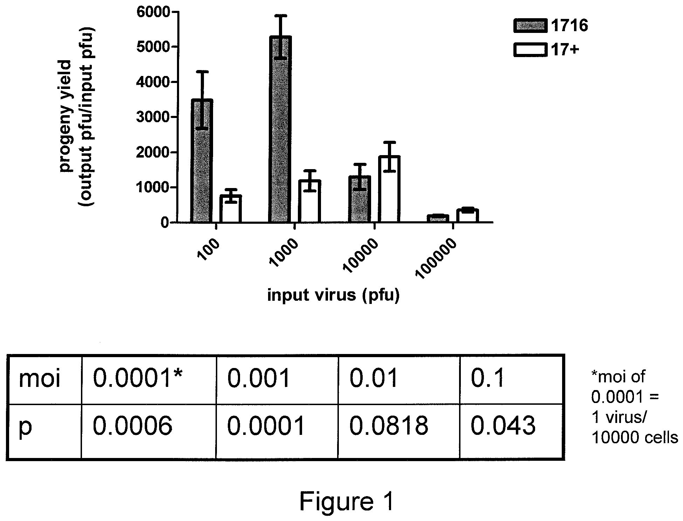

HSV1716 was found to replicate with high efficiency in HuH-7 and HepG2-luc2 cells in tissue culture (Figures 1-3).

At low multiplicities of infection (moi = number of input viruses/number of cells) in HuH-7 cells, HSV1716 replication gave higher progeny yields than parental wild-type HSV-1 17+ yields (Figure 1). Yields were similar at higher moi.

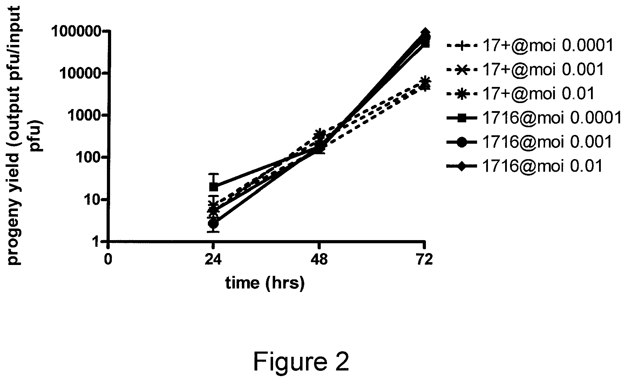

Similar levels of HSV-1 17+ and HSV1716 replication were found at 24 and 48 hrs, with a large increase in progeny production for HSV1716 between 48 and 72 hrs (Figure 2).

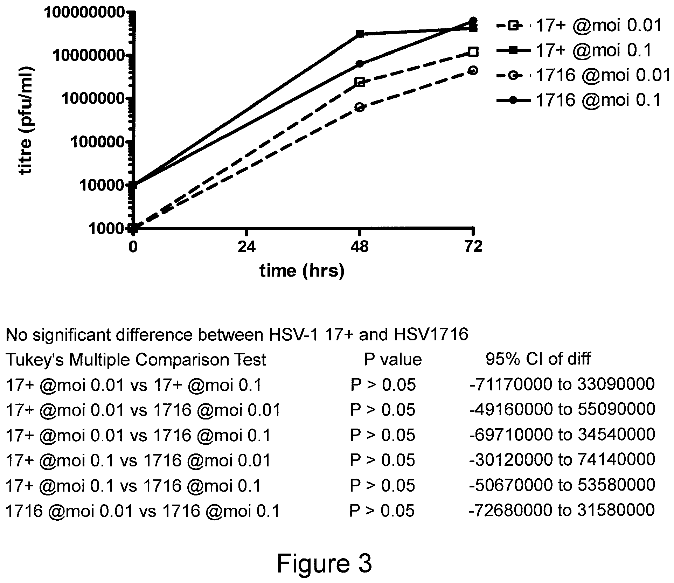

Progeny yields from HSV1716 infection of HepG2-luc2 cells were equivalent to those of wild-type HSV-1 17+, with no significant difference in yield between the viruses (Figure 3).

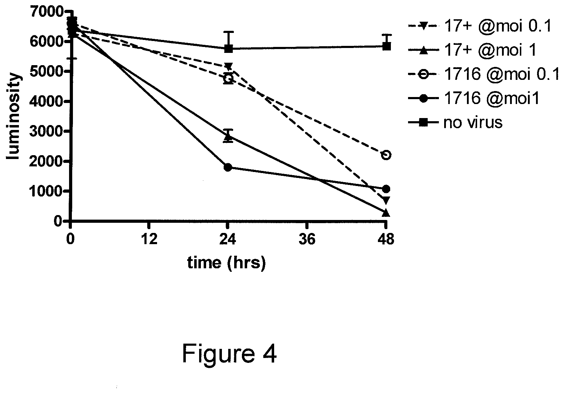

Luciferase activity decreased during HSV-1 17+ and HSV1716 infection of HepG2-luc cells (Figure 4). Efficient cell killing by virus correlates with loss of light production in HepG2-luc2 such that the loss of light output can be correlated with cell killing by HSV1716.

The results show that HuH-7 and HepG2-luc2 cells are excellent substrates for HSV1716 replication with high yields of progeny produced. HSV1716 was found to propagate in both cell types as efficiently as parental HSV-1 17+.

### Example 2 - Replication Kinetics of HSV17+ and HSV1716 in tumor cell lines

The following cell lines were used:
- Hepatocellular carcinoma (HCC): HuH7, HepG2-luc
- High Grade Glioma (HGG): U87
- Malignant Pleural Mesothelioma (MPM): SPC111
- Ovarian cancer: SKOV3
- Squamous cell carcinoma (SCC): A431

### 1) HuH7 (JCRB0403)

HuH-7 is a well-differentiated, hepatocyte-derived cellular carcinoma cell line that was originally taken from a liver tumor in a 57-year-old Japanese male. HuH-7 are epithelial-like tumorigenic cells which are able to form subcutaneous xenografts in nude mice. According to their entry in COSMIC HuH7 cells have mutated FAM123B and TP53 genes. HuH7 cells readily form subcutaneous xenografts in nude mice.

### 2) HepG2-luc (Caliper HT1080-luc2)

The Hep G2 cell line was isolated from a liver biopsy of a male Caucasian aged 15 years, with a well differentiated hepatocellular carcinoma. The cells secrete a variety of major plasma proteins e.g. albumin, alpha2-macroglobulin, alpha 1-antitrypsin, transferrin and plasminogen but Hepatitis B virus surface antigens have not been detected. They form very slow growing xenografts in nude mice.

HepG2-*luc*2 is a luciferase expressing cell line stably transfected with the firefly luciferase gene (*luc*2)*.* The cell line was established by transducing lentivirus containing luciferase 2 gene under the control of human ubiquitin C promoter.

### 3) U87 (aka U87MG, ECCAC - 89081402)

Epithelial like cells derived from a malignant glioma from a female patient by explant technique and reported to produce a malignant tumour consistent with glioblastoma in nude mice. Karyotype is 2n (=46). COSMIC (Catalogue Of Somatic Mutations In Cancer) entry indicates somatic mutations in CDKN2A, CDKN2C, CDKN2a(p14) and PTEN. U87 cells readily form subcutaneous xenografts in nude mice.

U87 cells are highly permissive for HSV1716 replication in tissue culture. After 72 hrs of replication HSV-1 17+ yields approximately 43100 +/- 13988 pfu progeny/input virus and HSV1716 yields 8806 +/- 2713 pfu progeny/input virus equivalent to a replication competence ratio (HSV1716 yield/HSV-1 17+ yield) of 0.2. Thus HSV1716 replication in U87 cells is impaired 5-fold compared to wild-type HSV-1 17+.

### 4) SPC111 (ECCAC 11120716)

SPC111 was derived from the pleural effusion of a 55-year old male patient, prior to treatment, with a known history of exposure to asbestos. The cells are epitheloid/mesenchymal. No entry in COSMIC.

SPC111 are permissive for HSV-1 17+ and HSV1716 replication. After 72 hrs of replication HSV-1 17+ yields approximately 11920 +/- 1002 pfu progeny/input virus and HSV1716 yields 2710 +/- 1343 pfu progeny/input virus equivalent to a replication competence ratio (HSV1716 yield/HSV-1 17+ yield) of 0.23 Thus HSV1716 replication in SPC111 cells is impaired 4-fold compared to wild-type HSV-1 17+.

### 5) SKOV3 - (aka SK-OV3, ECCAC -91091004)

Adherent, epithelial cells derived from the ascitic fluid from a 64 year old Caucasian female with an ovarian tumour that form moderately well-differentiated adenocarcinoma consistent with ovarian primary cells. Cells have a hypodiploid to hypotetraploid karyotype. COSMIC entry indicates somatic mutations in CDKN2A, CDKN2a(p14), MLH1, PIK3CA and TP53.

SKOV3 are permissive for HSV-1 17+ and HSV1716 replication. After 72 hrs of replication HSV-1 17+ yields approximately 27849 +/- 7511 pfu progeny/input virus and HSV1716 yields 913 +/- 299 pfu progeny/input virus equivalent to a replication competence ratio (HSV1716 yield/HSV-1 17+ yield) of 0.03 Thus HSV1716 replication in SKOV3 cells is severely impaired 30-fold compared to wild-type HSV-1 17+.

### 6) A431 (ECACC 85090402

A431 human squamous cell carcinoma cells are derived from an epidermal carcinoma of the vulva taken from an 85 year old female.

### Results

HuH7 cells were found to be highly permissive for HSV1716 replication both in tissue culture and in subcutaneous xenografts. After 72 hrs of replication HSV-1 17+ yields approximately 3250 +/- 814 pfu progeny/input virus and HSV1716 yields 28500 +/- 4815 pfu progeny/input virus equivalent to a replication competence ratio (HSV1716 yield/HSV-1 17+ yield) of 9. Thus HSV1716 replication in HuH7 cells is approximately 9-fold higher compared to wild-type HSV-1 17+.

HepG2-luc were also found to be fully permissive for HSV-1 17+ and HSV1716 replication. After 72 hrs of replication HSV-1 17+ yields approximately 4067 +/- 569 pfu progeny/input virus and HSV1716 yields 6003 +/- 987 pfu progeny/input virus equivalent to a replication competence ratio (HSV1716 yield/HSV-1 17+ yield) of 1.5. Thus HSV1716 replication in HepG2-luc cells is better compared to wild-type HSV-1 17+.

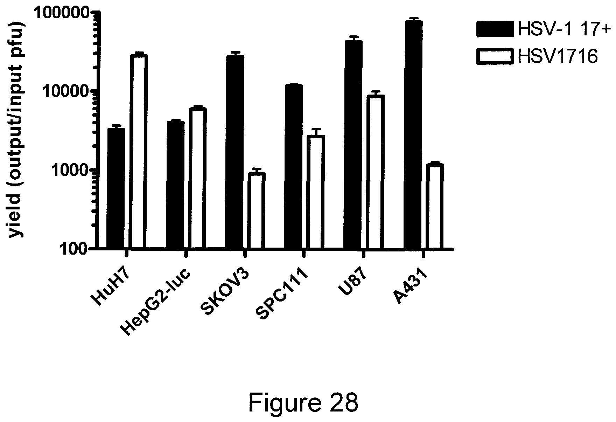

The progeny yields from replication of HSV1716 in both HuH7 and HepG2-luc cell lines indicated highly efficient propagation of virus in these HCC cell lines. Surprisingly, HSV1716 replication competence in both HuH7 and HepG2-luc cells is more potent than wild type HSV-1 17+ as higher progeny yields per input virus were obtained in both cell lines and the differences were significant with p=0.0001 and p=0.0145 for HuH7 and HepG2-luc respectively (Figure 28). This contrasts markedly with other human cancer cell lines, such as SKOV3 (ovarian), SPC111 (mesothelioma), U87 (high grade glioma) and A431 (SCC), in which HSV-1 17+ replication efficiency is much higher than that of HSV1716 (Figure 28).

As such, HSV1716 is shown to have better replication kinetics in HCC cells compared to the parent wild type strain HSV-1 17+. This is an unusual and surprising finding because an attenuated HSV would normally be expected to exhibit a reduction in replication kinetics compared to its corresponding parent strain, as is shown to be the case in the other tumor cell lines tested (ovarian, mesothelioma and high grade glioma). HCC tumors are normally slow growing which makes the result obtained even more surprising. Yet this finding indicates a marked potential efficacy of HSV1716 for the treatment of HCC.

### Example 3 - Demonstration of HSV1716 efficacy in a Hepatocellular Carcinoma xenograft model in nude mice

### HuH-7 xenografts - biodistribution of HSV1716

HuH-7 cells were implanted subcutaneously in nude mice and allowed to form subcutaneous tumors. Subcutaneous tumors developed in 10/10 mice. All animal procedures were performed under license from the UK Home Office. Female 6-8 week old athymic nude mice (Charles River Labs, Tranent, UK) were maintained under specific pathogen free conditions and all animal experiments were performed according to the appropriate UK guidelines.

### 1x10⁷ pfu HSV1716 was administered to all mice via a single tail vein injection.

Tumors/organs were harvested on days 1, 4, 7, 14 and 21 post-adminstration (n=2) and biodistribution of virus was analysed by titration.

HSV1716 was found to be almost exclusively localised to tumor tissue with high titres present throughout all samples. Most other tissues/organs were found to be free of virus at all times tested. In one mouse small amounts of virus were present in the liver on day 4 and lung on day 7. This may have been the result of residual inoculum, due to blood contaminating the tissue extracts or leakage into the circulation from the excess of virus in the tumor tissue.

### HSV1716 efficacy in HuH-7 xenografts - Part 1

30 mice were injected with HuH-7 cells, yielding 22 usable subcutaneous tumors.

Mice were separated in the following groups: No virus (n= 7), 1x10⁶ pfu HSV1716 (n=7) and 1x10⁷ pfu HSV1716 (n=8), where cGMP-like HSV1716 (Virttu Biologics Limited, Glasgow, UK) was administered via tail vein injection on days 1 and 4. Tumor growth and survival was monitored in all animals until animals were sacrificed. Titration of virus in tumors was performed from sacrificed HSV1716-treated mice.

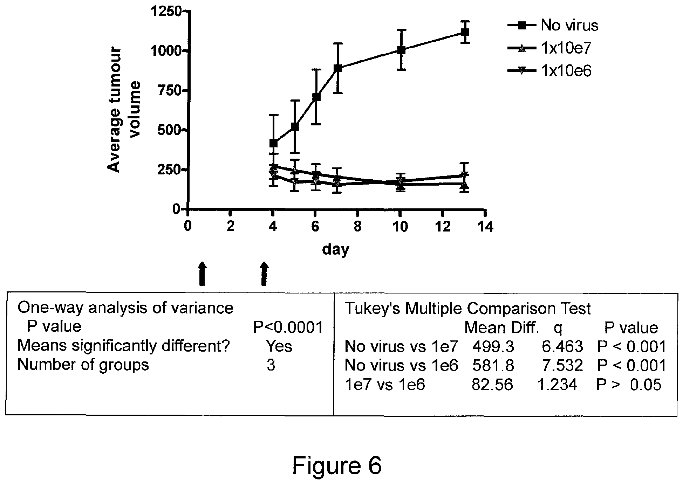

HuH7 tumors treated with HSV1716 at both doses have greatly reduced growth compared to untreated controls (Figure 6). The difference in tumor growth for untreated vs. treated was highly significant by ANOVA.

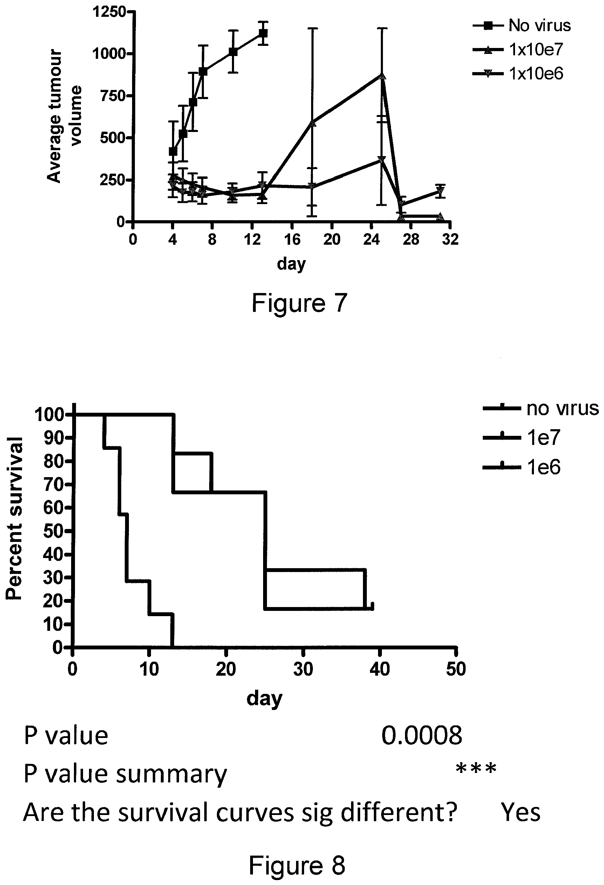

At the time of first injection there was heterogeneity in tumor sizes. Rapid tumor growth was observed after day 15 in mice which started the experiment with larger tumors (Figure 7). Beyond day 15 tumors in mice treated with 1x10⁷ pfu HSV1716 were seen to grow more rapidly, but this is considered to be due to the unequal distribution of tumor sizes at the start of the experiment with more mice with larger tumors in the 1x10⁷ group. After 28 days average tumor volume in both HSV1716 treated groups had returned to low levels (Figure 7).

The results show that intravenous HSV1716 is highly effective in treating a mouse model of HCC. Reduction in tumor growth and enhanced survival were both highly significant. High titres of HSV1716 in tumor extracts indicate prolific replication of virus in tumor cells. The lower dose of HSV1716 (1x10⁶ pfu) was at least as effective as the higher dose (1x10⁷ pfu)

### HSV1716 efficacy in HuH-7 xenografts - Part 2

Mice were injected with HuH-7 cells to generate subcutaneous tumor xenografts, as described above.

Mice were separated in the following groups: No virus (n= 5), 1x10⁵ pfu HSV1716 (n=6), 1x10⁶ pfu HSV1716 (n=6), and 1x10⁷ pfu HSV1716 (n=6) where cGMP-like HSV1716 (Virttu Biologics Limited, Glasgow, UK) was administered via tail vein injection on days 1, 14 and 29. Tumor growth and survival was monitored in all animals until animals were sacrificed. Titration of virus in tumors was performed from sacrificed HSV1716-treated mice.

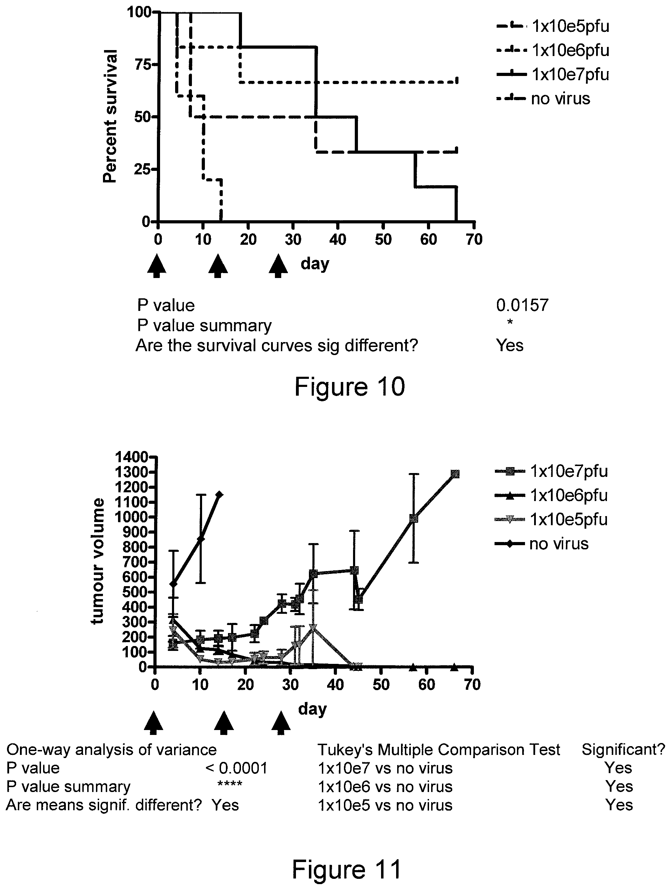

The results show that all control mice (injected with PBS only) were sacrificed by day 14 and that mice injected with HSV1716 had greatly improved survival (Figure 10). On day 66, when the experiment was stopped, 2/6 mice treated with 1x10⁵ pfu were cured, 4/6 mice treated with 1x10⁶ pfu were cured but there were no cures in 1x10⁷ pfu group.

Analysis of data from individual mice indicates that mice with large tumors at time of injection exhibited a poorer survival (3/3 receiving 1x10⁵ pfu HSV1716, 1/3 receiving 1x10⁶ pfu HSV1716 within 7 days) compared to those with smaller xenografts. Initially mice with larger tumors receiving 1x10⁷ pfu HSV1716 had slower growth and survived longer than mice with similar size tumors receiving 1x10⁵ HSV1716 and 1x10⁶ HSV1716 pfu but there were no cures and these tumors continued to grow. Most mice with smaller tumors at the time of first administration of 1x10⁵ or 1x10⁶ pfu HSV1716 were cured (5/6). Cure rates were higher in the 1x10⁶ group than in the 1x10⁵ group. No virus was found at the xenografts sites in cured mice.

The results show that intravenous administration of HSV1716 is treating HCC tumor xenografts. The reduction in tumor growth and enhanced survival were both highly significant. Very high titres of HSV1716 in tumor extracts indicate prolific replication of virus. 50% of mice were cured with 3 repeat doses at 14 day intervals. A dose of 1x10⁵ pfu HSV1716 was at least as effective as 1x10⁶ pfu HSV1716.

### Example 4 - Infection of HuH7 xenografts bv HSV1716 after intravenous administration

Mice were injected with HuH-7 cells to generate subcutaneous tumor xenografts, as described above.

### Experiment 1

HSV1716 (1x10⁷ pfu) was administered via tail vein injection to 4 mice with HuH7 xenografts. Mice were sacrificed 16, 24, 48 and 72 hours after HSV1716 injection. Tumors were extracted and virus titrated.

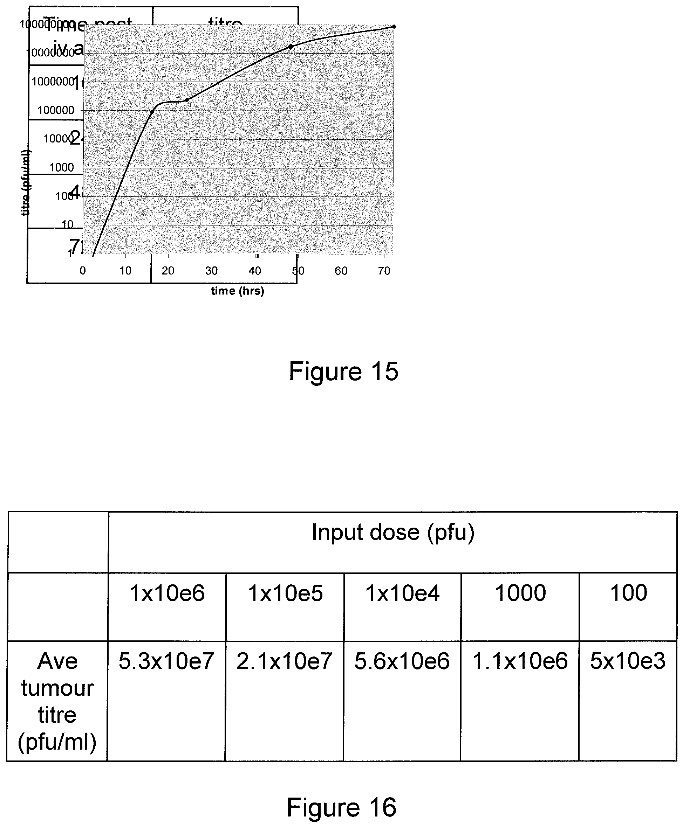

After 16 hours 1% of the input HSV1716 was seen to have localised to cells of the HuH-7 xenograft tumor (Figure 15). Within 24 hours a 100-fold increase in HSV1716 within cells of the tumor was observed (Figure 15). The HSV1716 replication kinetics were seen to be similar to those observed in vitro (Figure 15).

### Experiment 2

Mice (n=2) with HuH-7 xeongraft tumors were intravenously injected with increasing doses of HSV1716 (100, 1000, 1x10⁴, 1x10⁵ 1x10⁶ pfu). Tumors were harvested 72 hours after injection and analysed by titration.

Highly efficient localisation of HSV1716 to HuH-7 xenografts was observed, even at the lowest doses (Figure 16). Rapid replication of virus within the xenografts was also observed.

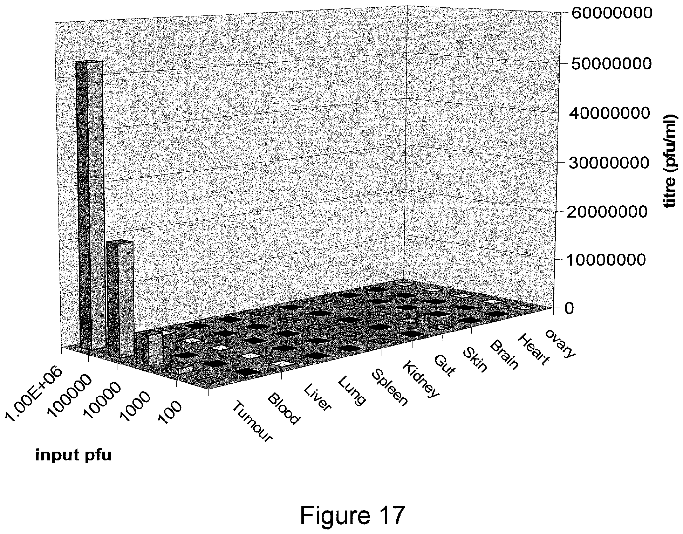

A biodistribution study showed HSV1716 to be present in tumor tissue only (Figure 17).

### Example 5 - HSV1716 in combination with doxorubicin in vivo

We investigated whether there is any loss of viral potency when HSV1716 is administered in combination with doxorubicin.

### Materials

- Mice with HuH-7 xenografts, as described above.
- HSV1716luc - a mutant of HSV1716 expressing the luciferase reporter gene.
- Doxorubicin salt (Caliper Life Sciences, Inc.)

By constructing an HSV1716 that conditionally expresses luciferase on viral replication, localisation of active virus in vivo can be detected by imaging for luminosity as a result of luciferase acting on the substrate luciferin.

### Methods

Nude mice with subcutaneous flank HuH-7 xenografts were injected with virus once the xenograft diameters were approximately 5 mm. All animals received 2x10⁶ pfu HSV1716luc.

Doxorubicin salt (specifically formulated for use in *in vivo* imaging) was made up in water at the time of injection. 10mg was resuspended in 5ml of water = 2mg/ml. 150µl was injected per mouse = 0.3mg. Mice weighed approximately 25g, thus equivalent dose is 12mg/kg. Equivalent to 36mg/m² (human dose is typically 40-60mg/m²).

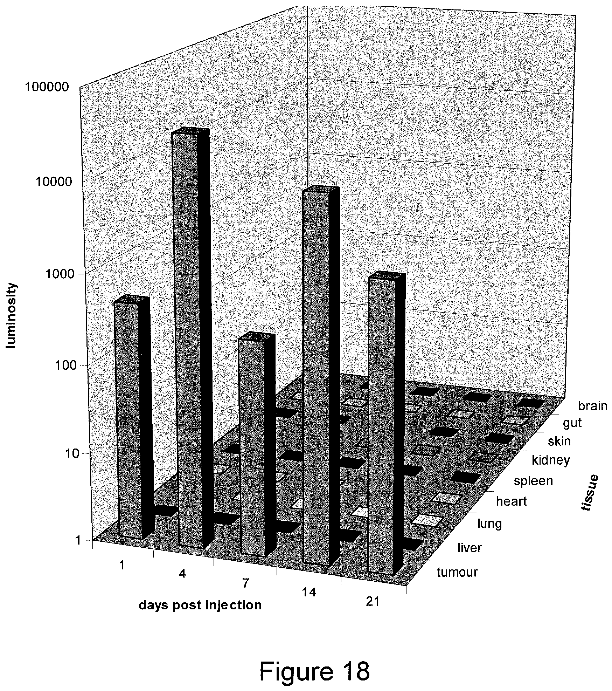

Following intravenous injection of 1x10⁷ pfu HSV1716luc into mice having subcutaneous HuH-7 xenograft luceiferase expression was detected by in vivo imaging analysis 72 hours after injection. Luciferase expression was found to be uniquely localised to tumor xenograft tissue, which is consistent with the biodistribution studies described in the previous examples. Luciferase expression remained localised to xenograft tumors (although of reduced size) after 21 days following injection (Figure 18).

### Experiment 1

Comparison of effect of:
1. 36mg/m² Doxorubicin mixed with 2x10⁶ pfu HSV1716luc injected intratumorally ("dox 1") and
2. PBS mixed with 2x10⁶ pfu HSV1716luc and injected intratumorally ("dox 2")

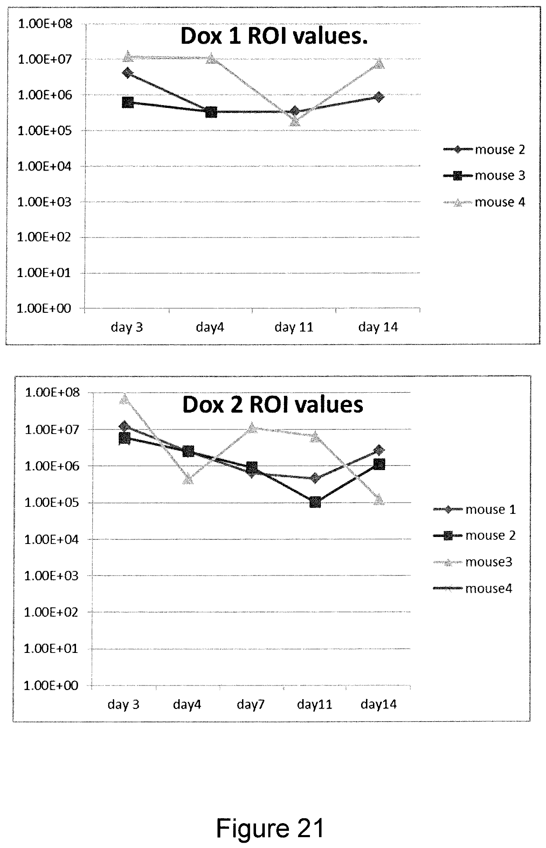

Light emissions from tumors injected with "dox 1" and "dox 2" were measured at days 3, 4, 11 and 14. Light emissions were similar for both groups (Figure 21) indicating that Doxorubicin has no apparent effect on HSV1716 replication following simultaneous intratumoral administration.

### Experiment 2

Comparison of effect of:
1. 2x10⁶ pfu HSV1716luc was injected intravenously ("dox 4"), and
2. 36mg/m² Doxorubicin was injected intratumorally, 2x10e6pfu HSV1716luc was injected intravenously ("dox 5")

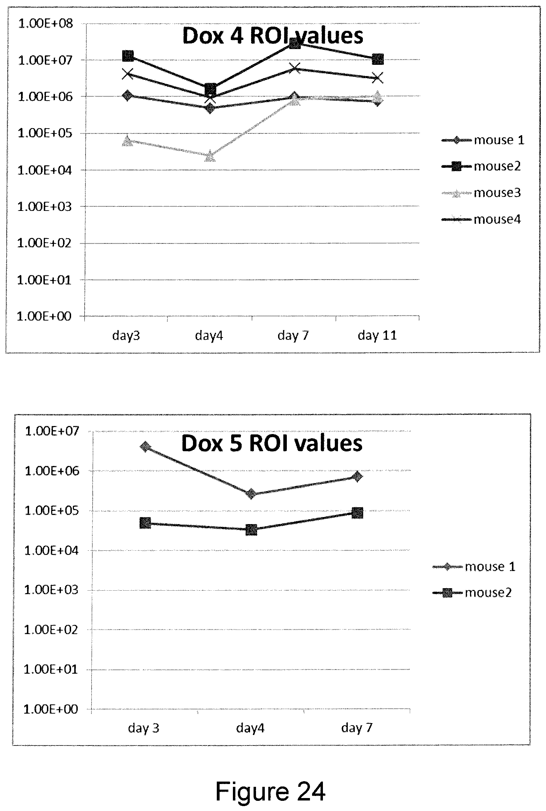

Light emissions from tumors injected with "dox 1" and "dox 2" were measured at days 3, 4, 7 and 11. Light emissions were similar for both groups (Figure 24) indicating that Doxorubicin has no apparent effect on HSV1716 replication following intravenous administration of HSV1716 and intratumoral administration of Doxorubicin.

### Experiment 3

36mg/m² Doxorubicin was injected intraperitoneally, and 2x10⁶ pfu HS1716luc was injected intratumorally ("dox 3").

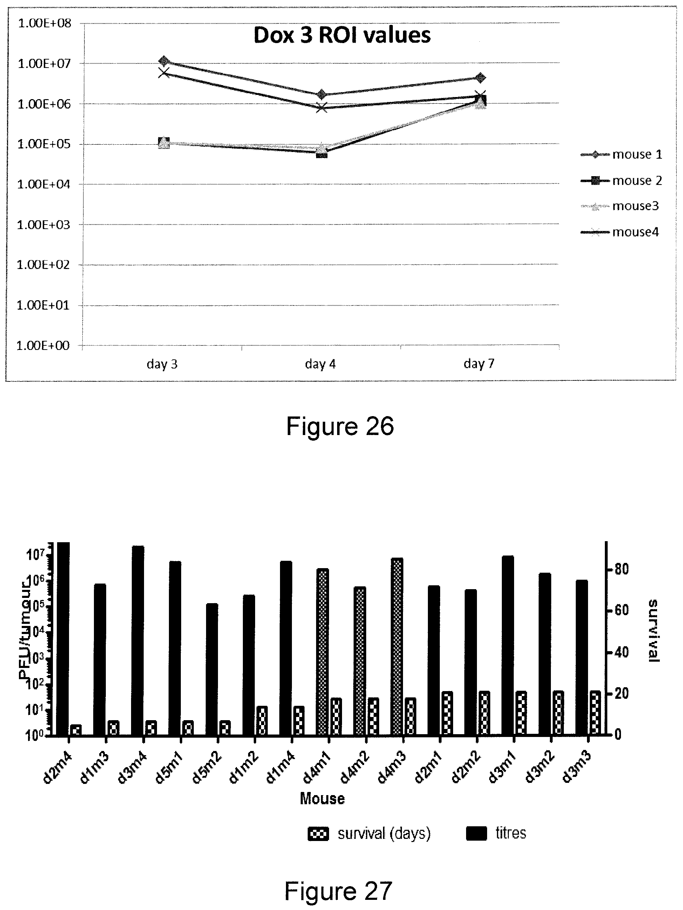

We observed that Doxorubcin injected intraperitoneally does not block replication of intratumoral HSV1716 (Figure 26).

For experiments 1-3 above, viral titre in tumor extracts was compared against survival (Figure 27). High virus titres were observed in all tumor extracts indicating that simultaneously administered doxorubicin does not affect HSV1716 replication in vivo.

### Example 6 - Phase I/II study of HSV1716 in HCC

A phase I/II study will be conducted investigating the safety, tolerability and biological effect of single and repeat intra-arterial injections of the selectively replication-competent herpes simplex virus HSV1716 in patients with unresectable hepatocellular carcinoma.

The study will comprise a dose escalation component. Delivery of virus will be into the hepatic artery using the Seldinger technique. CT scans will be conducted at baseline, day 28 and day 56. A biopsy will be taken at day 28 - one core from a lesion, one core from normal tissue.

### Stage 1

The primary objective of Stage 1 will be to assess the safety and tolerability of HSV1716 given by direct intra-arterial injection with TACE in patients with unresectable hepatocellular carcinomas.

The secondary objectives will be:
- to compare the safety information from Stage 1 with matched historical data from TACE
- to assess tumor response by measurement of tumor size by CT and the tumor marker α-FP
- to assess evidence of biological activity in pre- and post-treatment biopsy samples
- to assess the immune response to HSV1716 by measurement of circulating anti-HSV IgG and IgM
- to determine whether HSV1716 can be detected by PCR within the peripheral circulation after intra-arterial injection

### Stage 1

Stage 1 will be conducted at a single-centre, have an ascending dose design, and be open label. During their hepatic arteriography and prior to receiving the chemoembolisation agents, patients will receive a single intra-arterial injection of HSV1716 according to the following dosing regime:

| | Dose |
|---|---|
| Group 1 (3 patients) | 1 x 10⁶ pfu |
| Group 2 (3 patients) | 4 x 10⁶ pfu |
| Group 3 (3 patients) | 1 x 10⁷ pfu |

Delivery will be carried out during the TACE procedure by intra-arterial injection into a branch of the main hepatic artery supplying the tumor(s) and prior to the delivery of the TACE agents (typically, doxorubicin, lipiodol(contrast agent and vehicle for doxorubicin delivery into the tumor bed) and embolisation agent (for subsequent blockage of the tumor-feeding artery)).

### Stage 2

Stage 2 will be conducted at a single-centre, and have a randomised, controlled, two-arm open label study design.

30 patients will be randomised in a 2:1 ratio to receive HSV1716 plus TACE or TACE alone. In the treatment arm, patients will receive two doses of HSV1716 by intra-arterial injection. The optimal dose will be determined in Stage 1. The first dose will be administered during the TACE procedure. A 2 week interval between the first and second intra-arterial administration will be provided. In the control arm, patients will receive TACE as per standard of care.

### Example 7 - HSV1716 compatibility with delivery via TACE catheter and Doxorubicin (chemotherapeutic agent for TACE)

The aim of this experiment was to establish if there is any loss of viral titre using the materials and administration method to be used in the Phase I/II study described in Example 6 above.

### Materials

- Progreat Coaxial type catheter 2.7/2.9 fr, product code MC-PP27131.
- Boston scientific Floswitch Ref 44-200, UPN M001442000.
- CRU-01-A vial 47 1716 HSV1 2 x 10⁶ iu/ml (0.5ml used = 1x10⁶ iu)

### Methods

1. Progreat Coaxial type catheter and Boston scientific Floswitch are assembled in the class II hood
2. Pre-flush catheter with sterile saline (Baxters UKF 7114) to determine dead volume.
3. Transfer catheter tip to collection tube 1, 0.5ml of CRU-01-A is passed through the catheter using a 1ml luer syringe (Becton Dickinson ref 300912).
4. 5ml syringe (Becton Dickinson ref 302187) containing sterile saline is attached to floswitch.
5. A volume equal to dead volume of catheter is collected in tube 1 (dead volume).
6. The catheter tip is transferred to tube 2 and the next 0.5ml passed through the catheter is collected (virus flow through).
7. Transfer catheter tip to tube 3 and the next 0.5ml passed through the catheter is collected (post virus).
8. Transfer catheter tip to tube 4 and the remainder of the 5.0ml saline is passed through the catheter and collected (remaining NaCI).

| Tube | Vol (ml) | Titre (pfu/ml) | Sample type | TOTAL VIRUS OFF (pfu) | TOTAL VIRUS ON (pfu) |
|---|---|---|---|---|---|
| 1 | 0384 | 3.69x10⁵ | Dead volume | 2.52x10⁵ | |
| 2 | 0.551 | 1.01x10⁶ | Virus flow through | 5.57x10⁵ | |
| 3 | 0.906 | 5.40x10⁴ | 0.5ml post virus | 4.89x10⁴ | |
| 4 | 3.287 | 9.75x10² | Remaining NaCl | 3.20x103 | |
| | | | | **8.61x10⁵** | **9.7x10⁵** |

### Conclusion

From the data collected in this study, there was no significant reduction in viral titre using the equipment and delivery process.

Thus HSV1716 is fully compatible with the administration procedure and the TACE catheter.

### Example 8 - Further Demonstration of HSV1716 efficacy in a HepG2-luc Hepatocellular Carcinoma xenograft model in nude mice

The Hep-G2-luc cell line was chose as an alternative HCC cell line for verification of the results obtained using the HuH-7 HCC cell line reported in Examples 3 and 4 above. HepG2-luc cells are described above (see Examples 1 and 2).

### In-vitro assessment of HepG2-luc

1) Determine susceptibility of HepG2-luc cells to wild type and HSV1716 infection using luc assays and titration.
2) Characterise replication kinetics of HSV-1 17+ and HSV1716 in HepG2-luc cells.

### HepG2-luc cell killing by HSV1716 - microtitre plate assay.

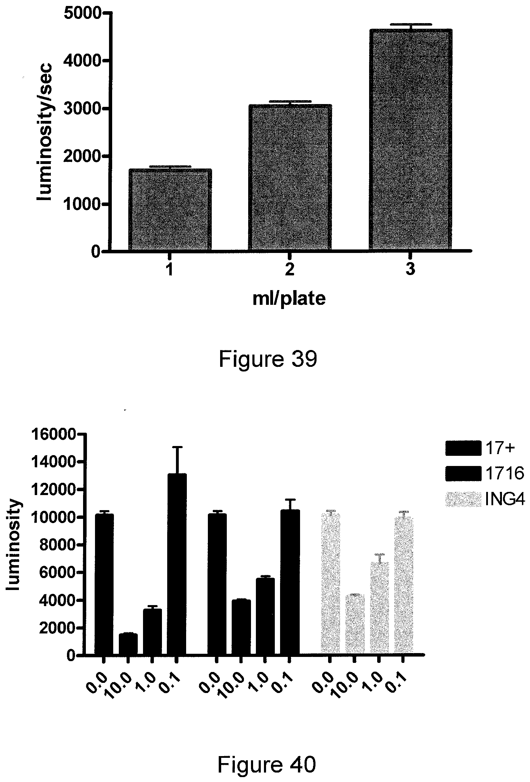

A T175 flask of HepG2-luc cells was split, cells were resuspended in 12ml and used to seed 96-well tissue culture quality microtitre plates. On a single 96-well microtitre plate HepG2-luc cells were plated out at densities equivalent to 1ml/plate, 2ml/plate or 3ml/plate. Eight wells were used for each density. After 24 hours in culture, luciferase substrate was added to each well (20µl working solution/well) and luciferase output was read immediately on a fluorescent plate reader (Perkin Elmer Victor X3) using a 0.1sec sampling time/well. The plate was shaken by the plate reader prior to measurement and the results presented in Fig 39.

Luminosity increased with increasing plating density of cells. A plating density of 2ml was selected as it generated sufficient signal strength in the optimum number of 96-well plates (6 per T175 flask). Cell counts by haemocytometer suggested that this was equivalent to at least 5000 cells/well although accurate counts were not possible because of the aggregates formed by the cells on splitting. Visual inspection of the wells suggested that this was a reasonably accurate figure.

HepG2 luc cells were plated out at 2ml cells/plate and after 24 hours HSV1716, HSV-1 17+ and HSV1716ING4* were added to the wells at moi 10, 1, and 0.1 (assuming 5000 cells/well). After a further 24 hours in culture luciferase activity was determined Figure 40. There is clear evidence of cell killing-mediated loss of light output for all three viruses at moi 10 and 1. After 24 hours of infection moi 0.1 did not demonstrate evidence of cell killing.

[*HSV1716ING4 has been deposited at the European Collection of Animal Cell Cultures, Vaccine Research and Production Laboratories, Public Health Laboratory Services, Porton Down, Salisbury, Wiltshire, SP4 0JG, United Kingdom under accession number 08021501 in accordance with the provisions of the Budapest Treaty, and is described in WO2008/099189.]

HepG2-luc cells were used to seed 96-well microtitre plates at 2ml/plate. After 24 hours in culture cells were infected with HSV1716 or HSV-1 17+ at 1 or 0.1 moi and luciferase activity determined at 24 and 48 hours post infection. Separate plates were used for the 24 and 48 hours post infection time points. Luciferase output over time is presented in Figure 4. Four luciferase readings were used per point.

Similar levels of moi-dependent cell killing were detected for HSV-1 17+ and HSV1716 with most cells dead by 48 hours. Visual inspection of the wells confirmed virus cytopathic effect (cpe) in most cells at this time. Luciferase outputs indicate that HSV1716 was as efficient as HSV-1 17+ at killing HepG2-luc cells.

### Multi-step growth curves in HepG2-luc cells.

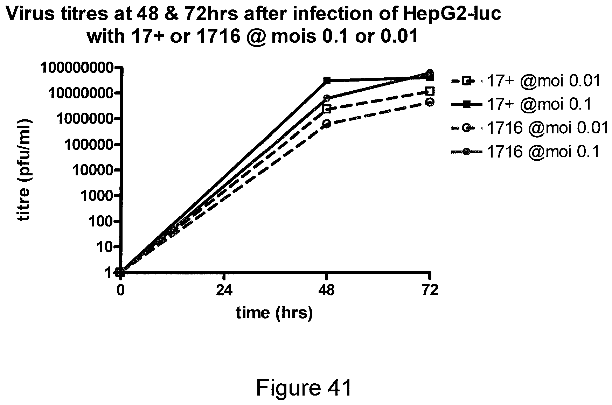

HepG2-luc cells were plated out in 60mm dishes and after 24 hours in culture cells were infected with HSV1716 or HSV-1 17+ at 0.1 and 0.01 moi in triplicate, (equivalent to 10000 or 100000 pfu of virus respectively added to plates). After 48 or 72 hours of infection, cells were harvested into the medium and total virus was titrated. Titres (pfu/ml) were derived from plaque counts and are presented in the Table below and in Figure 41.

**Table: Individual titres from HepG2-luc cells infected with 17+ or HSV1716 at moi 0.1 or 0.01 and harvested at 48 and 72 hours.**

| **Virus** | **moi** | **Time (hrs)** | **1** | **2** | **3** |
|---|---|---|---|---|---|
| HSV-1 17+ | 0.1 | 48 | 2.96x10e7 | 2.51x10e7 | 3.6x10e7 |
| HSV-1 17+ | 0.1 | 72 | 3.6x10e7 | 4.7x10e7 | 3.9x10e7 |
| HSV-1 17+ | 0.01 | 48 | 2.41x10e6 | 2.2x10e6 | 2.31x10e6 |
| HSV-1 17+ | 0.01 | 72 | 1.02x10e7 | 1.1x10e7 | 1.32x10e7 |
| HSV1716 | 0.1 | 48 | 7.2x10e6 | 5.4x10e6 | 6x10e6 |
| HSV1716 | 0.1 | 72 | 6.5x10e7 | 4.9x10e7 | 6.7x10e7 |
| HSV1716 | 0.01 | 48 | 5.7x10e5 | 5.9x10e5 | 6.7x10e5 |
| HSV1716 | 0.01 | 72 | 4.2x10e6 | 4.3x10e6 | 4.3x10e6 |

The HSV-1 17+ and HSV1716 titration data were analysed by ANOVA and there was no significant differences between them.

From these *in-vitro* experiments we concluded that HepG2-luc cells are excellent substrates for HSV1716 replication with high output of progeny and levels of replication efficiency similar to those of the wild-type HSV-1 17+ virus, and that HSV1716 replication in HepG2-luc cells can be monitored *in-vitro* by loss of luciferase activity.

### HepG2-luc animal experiments

### Phase 1: Intravenous or intratumoural injection in mice with HepG2-luc xenografts.

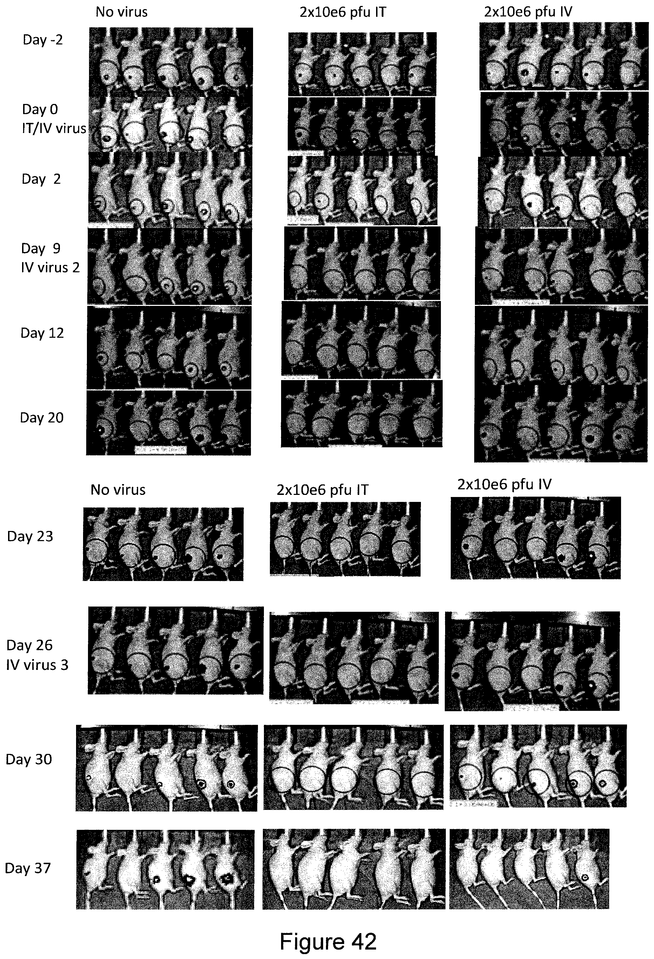

15 mice in total, 3 groups of 5 mice (intravenous, intratumoural, and control group). 15 mice were injected with cells harvested from 4x175cm² flasks. The HepG2-luc cell line expresses luciferase and can be imaged by IVIS. The injected cells rapidly established xenografts and luciferase expression was visualized at early times as the xenograft formed at the injection site (Figure 42). At day -2 (7 days post cell injection) light emissions from the xenografts were >10⁶ radiance and the mice were randomised into one of three groups which received no virus (an injection of PBS), intratumoural injection of 1x10⁶ pfu HSV1716 or an intravenous tail vein injection of 1x10⁶ pfu of HSV1716. By day 9 post virus injection there is no detectable luciferase signal in all five mice treated with HSV1716 intratumourally (Figure 42). At this time, 4/5 of the control mice had maintained strong luciferase signals which were consistently >10⁶ radiance and this continued until the end of the experiment on day 45 (Figure 42). The HepG2-luc cells did not establish a xenograft in one of the control mice indicating that the take rate for the cell line is 80%.

At day 9 all the IV-treated mice still had observable luciferase signals and received another IV injection (Figure 42). As the xenografts were small at this time it is possible that there was poor vascularisation to the xenograft area reducing or negating the efficiency of IV delivery. Thus, an additional virus injection was given on day 9 and a third on day 26. By day 37, 4/5 of the IV-treated group had no detectable luciferase signal indicating cures. The results of the experiment are summarised in the Table below and a selection of images are presented in Figure 42. All images in Figure 42 were adjusted to the same light scale so are directly compared.

**Table: Outcome for the HepG2-luc xenograft mice.**

| **Treatment** | **Outcome at end of experiment** |
|---|---|
| Intratumoural injection 1x10⁶ pfu | All 5 mice have no detectable luciferase signal = 5/5 cures |
| Intravenous injection 1x10⁶ pfu (x3) | 1 of 5 mice had detectable luciferase signal = 4/5 cures |
| No virus | 4 of 5 mice had detectable luciferase signal throughout = 0/4 cures |

### Phase 2: Efficacy of IT injections in mice with HepG2-luc xenografts

We investigated the effects of different doses of HSV1716 on HepG2-luc xenografts. 40 mice were injected with HepG2-luc cells. Approximately 1 confluent 175cm² flask was used per 4 mice. Mice were screened by IVIS and those not emitting a light signal were removed from the study. This reduced numbers from 40 to 28 giving four groups: 2x10⁴ pfu IT (n=7), 2x10⁵ pfu IT (n=5), 2x10⁶ pfu IT (n=8), and no virus (n=8). [IT = intratumoral administration]

### Results

Of the 7 mice injected with 2x10⁴ pfu HSV1716 intratumourally, 5 showed a reduction in luciferase levels by day 4 post injection. The remaining two mice were given a further intratumoural injection and 1 showed a decrease in luciferase level by 9 days post 2^{nd} injection. The remaining mouse was given a further intratumoural injection however this had no effect on the luciferase level. This mouse was euthanized at day 111. The tumour was excised and analysed for the presence of virus and no virus was detected. The most likely explanation is that the intratumoural injection had not been successful in this mouse.

5 mice received a single intratumoural injection of 2x10⁵ pfu HSV1716 and all showed complete loss of luciferase signal by day 14 post injection.

8 mice were injected intratumourally with 2x10⁶ pfu HSV 1716 and 6/8 showed complete loss of luciferase signal by day 4 post injection and 1 by day 14 post injection. The remaining mouse was given a further 2x10⁶ pfu HSV1716 by IT injection and showed a complete loss of luciferase expression within a further 9 days.

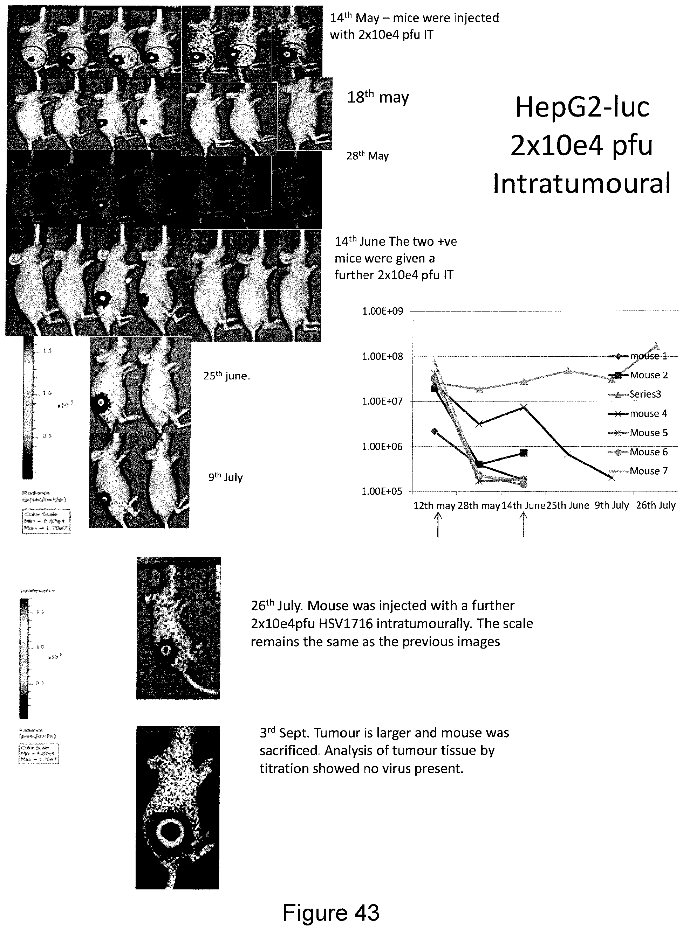

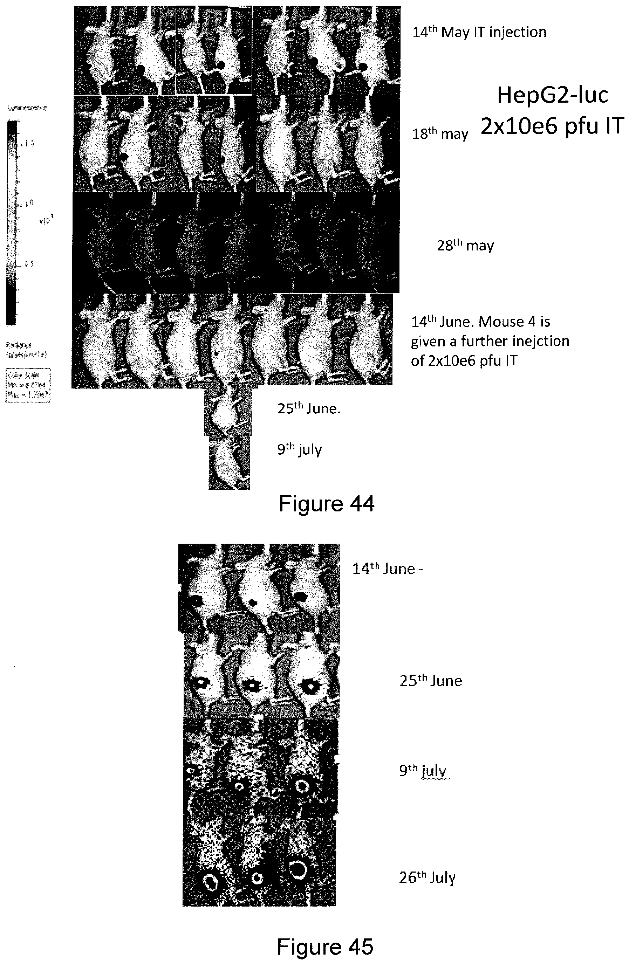

The 8 mice given no virus (an injection of equivalent amount of PBS) maintained strong luciferase expression throughout the experiment and this persisted long after luciferase expression had ceased in most of the treated mice. Results are summarised in the Table below and selected images from the groups are presented in Figs 43-45.

**Table: Outcome for the HepG2-luc xenograft mice in phase 2.**

| **Treatment** | **Outcome at end of experiment** |
|---|---|
| Intratumoural injection 2x10⁴ pfu | 6 of 7 mice have no detectable luciferase signal = 6/7 cures |
| Intratumoural injection 2x10⁵ pfu | 5 of 5 mice have no detectable luciferase signal = 5/5 cures |
| Intratumoural injection 2x10⁶ | 8 of 8 mice had no detectable luciferase signal = 8/8 cures |
| No virus | All 8 mice had detectable luciferase signal throughout = 0/8 cures |

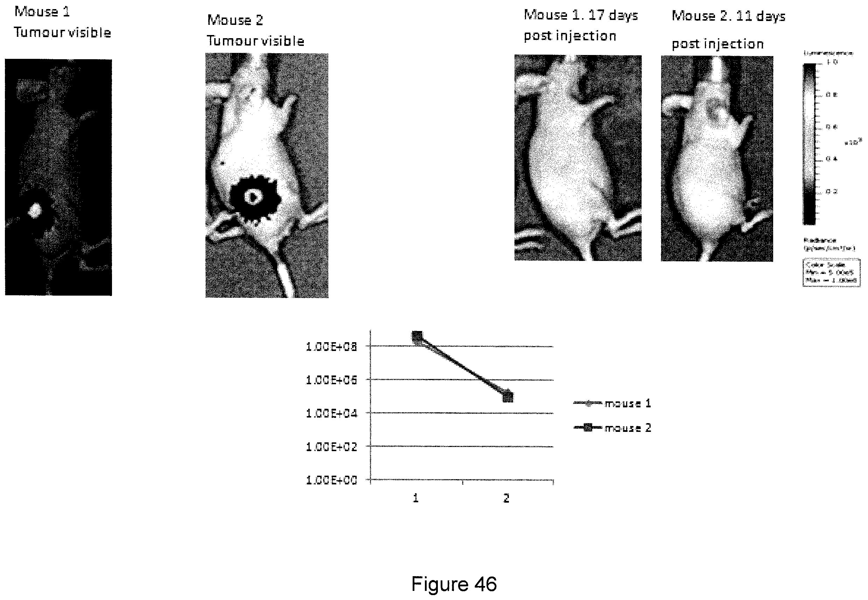

All mice received virus treatment at early stages of xenograft development and the ability of HSV1716 to treat long-established HepG2-luc xenografts was investigated using two untreated mice from the control group. At day 73 two of the control mice received 1x10⁶ pfu HSV1716 by IT injection. By days 11 or 17 post injection, the tumours had completely regressed and this was accompanied by complete loss of luciferase expression (Figure 46). Thus HSV1716 treatment was equally effective against early and late stage HepG2-luc xenografts in nude mice.

**Table: Overall summary of HepG2-luc in vivo results. Combined data from Phase 1 and 2 [*late stage HepG2-luc xenograft in control mice, **Cure was defined as complete and permanent loss of light emission from HepG2-luc xenograft].**

| **Treatment group** | **Number of mice** | **Cures**** |
|---|---|---|
| IV@ 1x10⁶ pfu | 5 | 4 |
| IT@ 1x10⁶ pfu | 5 | 5 |
| IT@ 2x10⁴ pfu | 7 | 6 |
| IT@2x10⁶ pfu | 5 | 5 |
| IT@2x10⁶ pfu | 8 | 8 |
| IT@ 1x10⁶ pfu* | 2 | 2 |
| No virus | 12 | 0 |

In summary, we found HepG2-luc to be a useful model to confirm our previous HuH7 xenograft data and tumour progression was measured very accurately using the IVIS. HepG2-luc cells were readily infected with HSV1716 and lytic replication coincides with loss of light output. HepG2-luc cells are very permissive for HSV1716 infection with high progeny yields. We observed impressive HSV1716 efficacy with mice bearing subcutaneous HepG2-luc xenografts.

*In-vivo,* HSV1716 completely kills the slow-growing xenografts formed by HepG2-luc cells in most animals tested. Particularly striking was the ability of a single injection to completely cure well-established tumours (n=2) and intravenous delivery of virus was successful in 4 of 5 mice even though the xenografts were small and probably had poor connection with the circulation.

### Example 9 - Materials for virus+drug combination studies

### Combination Studies

Cells were plated out in the internal 6x10 grid of a 96-well tissue culture plate at ∼5000 cells per well. Virus alone, drug alone or virus/drug combinations were added after 24 hours in culture in quadruplicate at least and, after a further 72 hours of incubation, their effects on cell death were determined using CytoTox-Glo Cytotoxicity Assay (Promega) which measures the activity of dead cell protease (DCP) released into the medium. Light emission from the DCP assay was detected using a Perkin Elmer 1420 multilabel counter Victor 3 in luminometer mode for 0.1 sec/well. Killing curves for virus alone and drug alone (GraphPad Prism 4) were used to determine IC50s and identify moi/drug concentrations for combination studies. Combination studies used 4 drug concentrations and two mois and were performed on a single plate with each plate repeated at least three times. Data was subjected to Chou-Talalay analysis to identify synergies and antagonisms using either CompuSyn software (Combosyn Inc.) or an in-house derived Chou-Talalay-based spreadsheet (a kind gift from Prof Tim Cripe).

### DCP Assay

Dead cell protease was assayed using the CytoTox-Glo Cytotoxicity kit from Promega. The kit provides a lumiogenic peptide substrate, AAF-Glo, to measure dead cell protease activity in the medium. As with LDH, dead cell protease is released from cells which have lost membrane integrity. The peptide substrate cannot cross the intact cell membrane of a live cell and will only be cleaved when dead cell protease has been released into the medium as cells die. The assay then uses the Ultra-Glo recombinant luciferase which can use the released aminoluciferin as substrate to generate a readily detectable luminescence signal. The DCP assay can potentially detect as few as 200-500 dead cells/well.

### HSV1716 for combination studies

An HSV1716 variant expressing green fluorescent protein is used for combination analysis. HSV1716gfp was produced from the parental HSV1716 by insertion of a CMV-gfp expression cassette in the UL-43 gene.

The virus stock titre was 1x10⁹ pfu/ml. The stock is diluted 1:100 in medium (0.5ml virus in 49.5ml) to give a 1x10⁷ pfu/ml stock and dispensed in 5ml aliquots for storage.

The 5ml of 1x10⁷ pfu/ml stock is diluted into 45ml medium to generate a 1x10⁶ pfu/ml stock, 6ml aliquots of which are stored for use in assays.

To dilute this 6ml to working strength (moi 0.5 and 0.05*):-
Moi 0.5 Add 5ml of the 1x10⁶ pfu/ml stock to 45ml medium (= 1x10⁵ pfu/ml), 50µl of this in the single well of a 96-well plate is equivalent to 5000 pfu/well.
Moi 0.05 Add 0.5ml of the 1x10⁶ pfu/ml stock to 49.5ml medium (= 1x10⁴ pfu/ml), 50µl of this in the single well of a 96-well plate is equivalent to 500 pfu/well.
* counts suggest between 8000-10000 cells /well for most cell types at time of infection. GFP was measured using a Perkin Elmer 1420 multilabel counter Victor 3 in FITC fluoremetry mode for 1 sec/well.

### In vivo tumour reduction studies

Female 6-8 week old athymic nude mice (Charles River Labs, UK) were maintained under specific pathogen free conditions. Actively growing HuH7 cells were harvested and, after re-suspension in PBS, 1x10⁶ cells per mouse were injected subcutaneously. Treatments were started when tumours reached 5mm in diameter. Mice were inspected daily, when tumour diameters reached 15mm they were sacrificed and the tumour xenograft removed for viral extraction. The viral load in tumours and various organs was assessed by plaque forming assay on Vero cells. Extracted intact tumours/organs were frozen immediately at -70°C and, after thawing, the tissues were mechanically homogenized in a Retsch homogenizer in 1ml PBS prior to titration.

### Example 10 - HSV1716 in combination with sorafenib

### HSV1716 in combination with sorafenib in vitro

HSV1716 was tested in combination with the tyrosine kinase inhibitor Sorafenib in 2 human HCC cell lines: HuH7 and HepG2-luc.

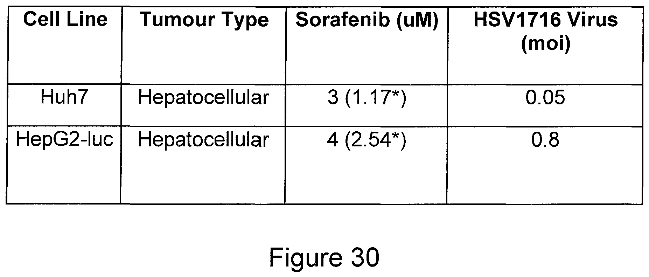

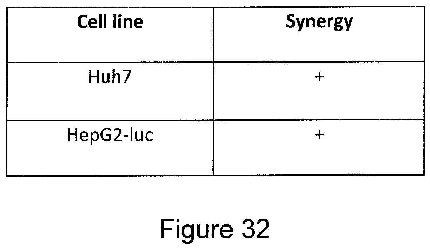

Sorafenib was purchased from Selleckchem UK (Cat#S1040, purity =100%) and was dissolved in DMSO to give a 20mM stock solution. Effects of virus alone, drug alone or virus/drug combinations on cell death were tested in culture after 72 hours of incubation using CytoTox-Glo Cytotoxicity Assay (Promega). Killing curves for virus alone, and sorafenib alone, were used to determine IC50s and identify moi/drug concentrations for combination studies (Fig 30). Combination studies used 4 sorafenib concentrations and two moi's and were performed on a single plate with each plate repeated at least three times. Data was subjected to Chou-Talalay analysis to identify synergies and antagonisms using CompuSyn software (Fig 32).

In-vitro combination studies of HSV1716 and sorafenib in 2 human HCC cell lines have shown that HSV1716 combines synergistically with sorafenib to enhance cell death in both HCC cell lines.

### HSV1716 in combination with sorafenib in vivo in subcutaneous flank HuH7 xenografts in nude mice

Sorafenib for IP injections was obtained from Selleckchem UK, (S1040), and was reported as 100% pure. The dose was 10mg/kg equivalent to 10µg/g or 250µg/mouse (25g). The 0.1ml intraperitoneal dose therefore required 250µg/0.1ml or 2.5mg/ml. 50mg sorafenib was dissolved in 0.5ml DMSO and this was added to 19.5ml PBS with 5% Tween80 and 5% PEG400 (both v/v). This is equivalent to c4mM sorafenib. HSV1716gCluc which expresses luciferase from the HSV-1 gC promoter. The stock virus solution was 8x10⁷ pfu/ml and 0.125ml virus stock were added to 10ml compound sodium lactate with 10% glycerol to give the injection solution at a titre of 1x10⁶ pfu/ml. 0.1ml injected it/mouse and is equivalent to 1x10⁵ pfu.

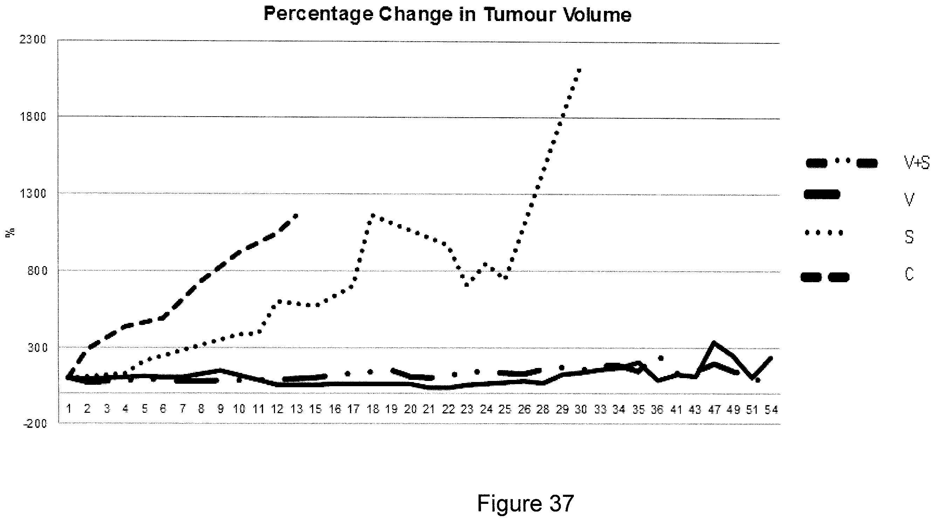

In vivo combination with sorafenib and HSV1716 was analysed using the HuH7 subcutaneous xenograft HCC model in nude mice. Groups were - no treatment (n=10), sorafenib (n=6), HSV1716 (n=10), HSV1716 + sorafenib (n=12). HSV1716gC-luc at 1x10⁵ pfu was given on days 1, 3 and 9 and sorafenib at 10mg/kg (Liu et al Cancer Res 2006;66:11851-11858), was administered IP (Wang et al., Cancer Biol & Therapy 2011; 12:3, 229-238) every 2nd day from day 2 on 9 occasions. Virus was monitored by IVIS and tumour growth (Fig. 37) and survival (Fig. 38) were recorded.

Excellent inhibition of growth and prolonged survival in mice treated with virus alone. Growth was inhibited by sorafenib during treatment and the mice survived longer than controls. For xenograft growth and mouse survival, HSV1716+ sorafenib was equivalent to virus alone although there was a trend to improved survival in the HSV1716+sorafenib-treated mice vs HSV1716-treated alone when mice were receiving sorafenib between days 2-18 (100% vs 80%).

The combination of oncolytic virus and sorafenib is shown to extend the period in which no mice died from about 4 days with sorafenib or about 7 days with virus only to about 19 days with virus and sorafenib (Figure 38). This indicates an extension of the survival benefit provided by sorafenib.

### Example 11 - HSV1716 in combination with doxorubicin in vitro

HSV1716 and doxorubicin combinations in 2 human HCC cell lines: HuH7, and HepG2-luc were tested.

Doxorubicin was purchased from Selleckchem UK (Cat # S1208, purity =99.01 %) and was dissolved in DMSO to give a 20mM stock solution. Effects of virus alone, drug alone, or virus/drug combinations on cell death were tested in culture after 72 hours of incubation using CytoTox-Glo Cytotoxicity Assay (Promega). Killing curves for virus alone and doxorubicin alone were used to determine IC50s and identify moi/drug concentrations for combination studies. Combination studies used 4 doxorubicin concentrations and two moi's and data was subjected to Chou-Talalay analysis to identify synergies and antagonisms using CompuSyn software (Combosyn Inc.).

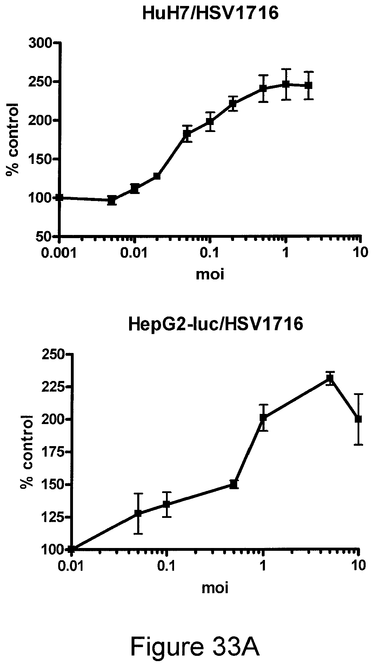

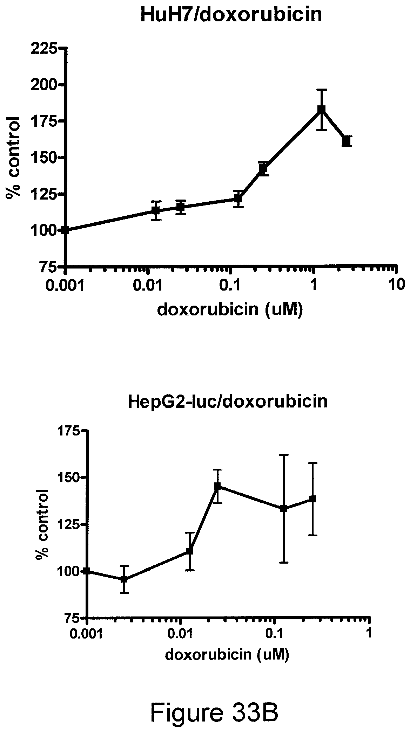

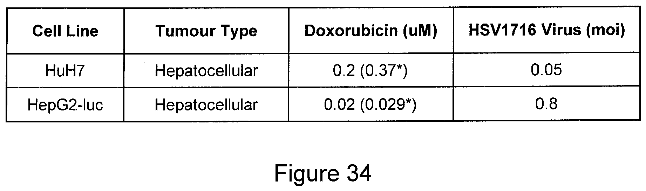

IC 50 data for HSV1716 and Doxorubicin are shown in Figure 33 and the calculated IC50's are shown in Figure 34.

In conclusion, in-vitro combination studies of HSV1716 and doxorubicin in 2 human HCC cell lines have shown that HSV1716 combines synergistically with doxorubicin to enhance cell death in HuH7 and HepG2-luc HCC cell lines.

## Claims

1. HSV1716 for use in a method of treating a hepatocellular carcinoma, the method comprising intravenous or intra-arterial administration of HSV1716, wherein the hepatocellular carcinoma is **characterised in that** HSV1716 propagation in cells of the hepatocellular carcinoma *in vitro* is at least as efficient as propagation of HSV-1 17+.

2. HSV1716 for use in a method of treating a hepatocellular carcinoma according to claim 1, wherein the method further comprises administration of doxorubicin.

3. HSV1716 for use in a method of treating a hepatocellular carcinoma according to claim 1, wherein the method further comprises simultaneous or sequential intra-arterial or intravenous administration of doxorubicin.

4. HSV1716 for use in a method of treating a hepatocellular carcinoma according to claim 3, wherein administration of the oncolytic herpes simplex virus and doxorubicin is to the hepatic artery.

5. HSV1716 for use in a method of treating a hepatocellular carcinoma according to claim 1, wherein the method further comprises administration of sorafenib.

6. HSV1716 for use in a method of treating a hepatocellular carcinoma according to claim 1, wherein the method further comprises oral administration of sorafenib.

7. HSV1716 for use in a method of treating a hepatocellular carcinoma according to any one of claims 1 to 6, wherein administration of the oncolytic herpes simplex virus is to the hepatic artery.

8. A pharmaceutical composition comprising HSV1716 and a chemotherapeutic agent, wherein the chemotherapeutic agent is doxorubicin or sorafenib.

9. A kit comprising a predetermined amount of HSV1716 and a predetermined amount of chemotherapeutic agent, wherein the chemotherapeutic agent is doxorubicin or sorafenib.

10. Products containing therapeutically effective amounts of:
(i) HSV1716, and
(ii) Doxorubicin and/or Sorafenib
for simultaneous or sequential use in a method of medical treatment, preferably treatment of primary liver cancer.

## Patentansprüche

1. HSV1716 zur Verwendung in einem Verfahren zum Behandeln eines Leberzellkarzinoms, wobei das Verfahren eine intravenöse oder intraarterielle Verabreichung von HSV1716 umfasst, wobei das Leberzellkarzinom **dadurch gekennzeichnet ist, dass** eine HSV1716-Verbreitung in Zellen des Leberzellkarzinoms *in vitro* wenigstens so effizient ist wie eine Verbreitung von HSV-1 17+.

2. HSV1716 zur Verwendung in einem Verfahren zum Behandeln eines Leberzellkarzinoms nach Anspruch 1, wobei das Verfahren ferner eine Verabreichung von Doxorubicin umfasst.

3. HSV1716 zur Verwendung in einem Verfahren zum Behandeln eines Leberzellkarzinoms nach Anspruch 1, wobei das Verfahren ferner eine gleichzeitige oder sequentielle intraarterielle oder intravenöse Verabreichung von Doxorubicin umfasst.

4. HSV1716 zur Verwendung in einem Verfahren zum Behandeln eines Leberzellkarzinoms nach Anspruch 3, wobei die Verabreichung des onkolytischen Herpes-simplex-Virus und von Doxorubicin in die Leberarterie erfolgt.

5. HSV1716 zur Verwendung in einem Verfahren zum Behandeln eines Leberzellkarzinoms nach Anspruch 1, wobei das Verfahren ferner die Verabreichung von Sorafenib umfasst.

6. HSV1716 zur Verwendung in einem Verfahren zum Behandeln eines Leberzellkarzinoms nach Anspruch 1, wobei das Verfahren ferner eine orale Verabreichung von Sorafenib umfasst.

7. HSV1716 zur Verwendung in einem Verfahren zum Behandeln eines Leberzellkarzinoms nach einem der Ansprüche 1 bis 6, wobei die Verabreichung des onkolytischen Herpes-simplex-Virus in die Leberarterie erfolgt.

8. Pharmazeutische Zusammensetzung, umfassend HSV1716 und ein chemotherapeutisches Mittel, wobei das chemotherapeutische Mittel Doxorubicin oder Sorafenib ist.

9. Satz, umfassend eine vorgegebene Menge an HSV1716 und eine vorgegebene Menge des chemotherapeutischen Mittels, wobei das chemotherapeutische Mittel Doxorubicin oder Sorafenib ist.

10. Erzeugnisse, enthaltend therapeutisch wirksame Mengen von:
(i) HSV1716 und
(ii) Doxorubicin und/oder Sorafenib
zur gleichzeitigen oder sequentiellen Verwendung in einem Verfahren einer medizinischen Behandlung, bevorzugt einer Behandlung von primärem Leberkrebs.

## Revendications

1. HSV1716 destiné à être utilisé dans un procédé de traitement d'un carcinome hépatocellulaire, le procédé comprenant l'administration intraveineuse ou intra-artérielle de HSV1716, dans lequel le carcinome hépatocellulaire est **caractérisé en ce que** la propagation du HSV1716 dans les cellules du carcinome hépatocellulaire *in vitro* est au moins aussi efficace que la propagation du HSV-1 17+.

2. HSV1716 destiné à être utilisé dans un procédé de traitement d'un carcinome hépatocellulaire selon la revendication 1, dans lequel le procédé comprend en outre l'administration de doxorubicine.

3. HSV1716 destiné à être utilisé dans un procédé de traitement d'un carcinome hépatocellulaire selon la revendication 1, dans lequel le procédé comprend en outre l'administration intra-artérielle ou intraveineuse simultanée ou séquentielle de doxorubicine.

4. HSV1716 destiné à être utilisé dans un procédé de traitement d'un carcinome hépatocellulaire selon la revendication 3, dans lequel l'administration du virus oncolytique de l'herpès simplex et de la doxorubicine se fait dans l'artère hépatique.

5. HSV1716 destiné à être utilisé dans un procédé de traitement d'un carcinome hépatocellulaire selon la revendication 1, dans lequel le procédé comprend en outre l'administration de sorafénib.

6. HSV1716 destiné à être utilisé dans un procédé de traitement d'un carcinome hépatocellulaire selon la revendication 1, dans lequel le procédé comprend en outre l'administration orale de sorafénib.

7. HSV1716 destiné à être utilisé dans un procédé de traitement d'un carcinome hépatocellulaire selon l'une quelconque des revendications 1 à 6, dans lequel l'administration du virus oncolytique de l'herpès simplex se fait dans l'artère hépatique.

8. Une composition pharmaceutique comprenant HSV1716 et un agent chimiothérapeutique, dans laquelle l'agent chimiothérapeutique est la doxorubicine ou le sorafénib.

9. Une trousse comprenant une quantité prédéterminée de HSV1716 et une quantité prédéterminée d'agent chimiothérapeutique, dans laquelle l'agent chimiothérapeutique est la doxorubicine ou le sorafénib.

10. Des produits contenant des quantités thérapeutiquement efficaces de :
(i) HSV1716, et
(ii) doxorubicine et/ou sorafénib
pour une utilisation simultanée ou séquentielle dans un procédé de traitement médical, de préférence le traitement du cancer primitif du foie.
